# EUROPÄISCHE PATENTANMELDUNG

(11) **EP 3 025 701 A1**
(43) Veröffentlichungstag der Anmeldung: **01.06.2016**
(21) Anmeldenummer: 15196467.3
(22) Anmeldetag: 26.11.2015
(51) Int. Cl.: A61K 8/25, A61Q 17/04, C01B 33/027, C01B 33/029

(54) **NANOKRISTALLINE SILICIUMPULVER, VERFAHREN ZU DESSEN HERSTELLUNG ALS AUCH DEREN VERWENDUNG**

(30) Priorität: 28.11.2014 EP 14195305
(71) Anmelder: Evonik Degussa GmbH, 45128 Essen (DE)
(72) Erfinder: LANG, Jürgen Erwin, 76229 Karlsruhe (DE); RAULEDER, Hartwig, 79618 Rheinfelden (DE); UEHLENBRUCK, Goswin, 61440 Oberursel (DE)

(57) **Zusammenfassung**

Die Erfindung betrifft höchstreine, nanokristalline Siliciumpulver, umfassend kristalline Submicropartikel, die optional auf einer Siliciumlegierung mit Elektronendonatoren- und/oder Elektronenakzeptoren basieren und/oder eine Zone einer entsprechenden Legierung aufweisen sein können. Ferner wird ein Verfahren zur Herstellung der Siliciumpulver sowie die Verwendung eines Reaktors zur Herstellung der Siliciumpulver offenbart. Die erfindungsgemäßen Siliciumpulver können vorzugsweise zur Herstellung von Halbleiterausgangsstoffen, Halbleitern, Thermoelementen zur Energierückgewinnung aus Abwärme, insbesondere von hochtemperaturstabilen Thermoelementen verwendet werden.

## Beschreibung

Die Erfindung betrifft reine, nanokristalline Siliciumpulver, umfassend kristalline Submicropartikel, die optional auf einer Siliciumlegierung mit Elektronendonatoren- und/oder Elektronenakzeptoren basieren und/oder eine Zone einer entsprechenden Legierung aufweisen können. Ferner wird ein Verfahren zur Herstellung der Siliciumpulver sowie die Verwendung eines Reaktors zur Herstellung der Siliciumpulver offenbart. Die erfindungsgemäßen Siliciumpulver können vorzugsweise zur Herstellung von Halbleiterausgangsstoffen, Halbleitern, Thermoelementen zur Energierückgewinnung aus Abwärme, insbesondere von hoch-temperaturstabilen Thermoelementen verwendet werden.

In der Solar- und Halbleiterindustrie sowie der LED- und Display-Herstellung besteht ein großer Bedarf an hochreinen Verbindungen, die zur Herstellung und Nutzung der Technologien benötigt werden. Den bekannten Prozessen zur Herstellung von Silicium gemeinsam sind hohe Rohstoffkosten, ein hoher Energieeintrag und eine für die vorgenannten technischen Anwendungsfelder nicht ausreichende Reinheit der erhaltenen Produkte.

Vielen der heutigen Industrieapplikationen von Silicium sind in der Regel die sehr hohen Reinheitsanforderungen gemeinsam. Daher darf die Verunreinigung der umzusetzenden Silane oder Halogensilane höchstens im Bereich von wenigen mg/kg (ppm-Bereich) und für spätere Anwendungen in der Halbleiterindustrie für einzelne Elemente im Bereich von wenigen µg/kg (ppb-Bereich) liegen.

Aufgabe ist die energieeffiziente Herstellung von kristallinen Si-Partikeln sowie die Bereitstellung eines Verfahrens zur einfachen und ökonomischen Gewinnung von hochreinem Silicium, das erfindungsgemäß kristallin oder polykristallin vorliegen sollen.

Des Weiteren bestand die Aufgabe einen Prozess zu finden, der kontinuierlich oder satzweise hohe Durchsätze der herzustellenden Verbindungen erlaubt. Eine weitere Aufgabe bestand darin die Partikel darüber hinaus definiert mit Elektronenakzeptoren oder Elektronendonatoren zu versehen, die das technische Anwendungsprofil der Silicium enthaltenden Partikel deutlich erweitert. So sollen Silicium enthaltende Partikel hergestellt werden, die in Thermoelementen oder in hochtemperaturstabilen Folien, Schichten, Bändern oder Bauteilen als umweltfreundliche Verbindungen zum Einsatz kommen können. So soll ein Verfahren entwickelt werden, dass auch ohne die Verwendung von Tetrachlorsilan wirtschaftlich ist.

Des Weiteren sollen die spektrale Eigenschaften der Silicium enthaltenden Partikel über die Verteilung der Elektronenakzeptoren und/oder Elektronendonatoren im jeweiligen Partikel und/oder auf dem jeweiligen Partikel einstellbar sein. Beispielsweise sollen die, wärmeleitenden Eigenschaften für die Thermoelektrik bspw. verringert werden oder im Zusammenhang mit einer verbesserten Transparenz sollen die wärmeleitenden Eigenschaften verbessert werden, um Wärme besser aus elektronischen Geräten abzuleiten. Ferner sollen die Transparenz, die Eigenfarbe, die elektrischen Eigenschaften der kristallinen Partikel definiert über das Verfahren und die gewählte Prozessführung einstellbar sein. Das erfindungsgemäße Verfahren soll zudem wirtschaftlicher sein als bekannte Verfahren, in dem die nicht umgesetzten Reaktionsprodukte oder die verwendeten inerten Gase ökonomischer wiederverwendet werden können.

Die Aufgaben konnten in überraschender Weise dadurch gelöst werden, dass die oben genannten Siliciumverbindungen durch Umsetzung von Monosilan oder höheren H-Silanen, wie der Formel MeHₙ oder Me₂H₂ₙ₋₂, wobei Me für Silicium und n für eine ganze Zahl steht, Halogensilanen, höheren Halogensilanen, wobei die Halogen Silane Wasserstoff enthalten können oder perhalogniert sein können, oder Alkoxysilanen mit C oder N oder Ge enthaltenden Spezies in einem thermischen Prozess, ggf. kombiniert mit einem Plasmaprozess oder in einem reinen Plasmaprozess umgesetzt werden. Höhere H-Silane oder Halogensilane, wie Chlorsilane, werden auch als Polysilane oder Polychlorsilane bezeichnet. Die höheren H-Silane oder höheren Chlorsilane werden vor Ihrer Umsetzung in die Gasphase überführt. Das Halogen kann ausgewählt sein aus Fluor, Chlor, Brom oder Iod, bevorzugt ist Chlor.

Gelöst werden die Aufgaben durch das erfindungsgemäße Siliciumpulver nach Anspruch 1 und das erfindungsgemäße Verfahren nach Anspruch 8 und durch Verwendung eines Freiraum-Reaktor nach Anspruch 14. Bevorzugte Ausführungsformen sind in den Unteransprüchen und in der Beschreibung erläutert.

So benötigen übliche konventionelle Verfahren, die auf einer Umsetzung von SiCl₄ basieren von 40 und mehr als 100 kWh/kg. Die erfindungsgemäßen Verfahren beruhen vorzugsweise auf einer Umsetzung von Monosilan mit einem Energiebedarf von weniger als 40 kWh/kg.. Des Weiteren wird für die Umsetzung im Plasma der Energiebedarf weiter reduziert auf kleiner 1,4 kWh/kg.

Erfindungsgemäß wird ein großtechnisches, industrielles, vorzugsweise kontinuierliches Verfahren zur Herstellung von Siliciumpulvern umfassend Submicropartikel und optional Cluster der Submicropartikel in Form von Marcropartikeln bereitgestellt. Bevorzugte Siliciumpulver umfassen Silicium-Pulver, die vorteilhaft Zonen, beispielsweise zumindest teilweise Beschichtungen mit Siliciumlegierungen mit Elektronenakzeptoren- oder Elektronendonatoren und/oder intrinsische Zonen von Siliciumlegierungen aufweisen können. Die Umsetzung, insbesondere umfassend die Zersetzung von gasförmigen Siliciumverbindungen und die Bildung der Siliciumpartikel oder der beschichteten Siliciumpartikel, kann bei Temperaturen ab 600 °C, bevorzugt ab 800 bis 1800 °C zur Herstellung kristalliner Pulver erfolgen. Zur Herstellung kristalliner Siliciumpulver werden definierte Kontaktzeiten, vorzugsweise bei Temperaturen unter 1800 °C, insbesondere um 900 bis 1600 °C gewählt. Die Abscheidung der Siliciumpulver erfolgt in einer kühleren Zone des Reaktors. Bevorzugte Kontaktzeiten zur Zersetzung und Abscheidung der Submicropartikel und optional Macropartikel liegen bei 10 bis 4000 Millisekunden.

Die Aufgaben konnten nach einer Alternative überraschend gelöst werden durch eine Behandlung der Siliciumverbindung im nicht-thermischen Plasma, insbesondere in einem nicht-thermischen Plasma erzeugt durch eine Gasentladung, auf einen die erforderlichen Reaktionskomponenten enthaltenden Massestrom.

Gegenstand der Erfindung ist die Erzeugung von reinen, gegebenenfalls mit Donatoren oder Akzeptoren legierten Siliciumpulvern unter thermischen Bedingungen sowie auch die Gewinnung von reinen, gegebenenfalls legierten Siliciumpulvern durch Reaktion im Plasma. Gegenstand der Erfindung sind auch Siliciumpulver umfassend mindestens ein Submicropartikel mit einer Siliciumlegierung mit größer gleich 10²¹ Atomen/cm³ ausgewählt aus mindestens einem Elektronendonator und Elektronenakzeptor.

Weiter ist es bevorzugt, wenn die Submicropartikel im Wesentlichen aus einer monokristallinen Phase umfassend Silicium bestehen.

Die Erfindung betrifft auch ein Verfahren zur Gewinnung von hochreinen, mit Siliciumlegierungen umfassend (Elektronen-)Donatoren oder (Elektronen-)Akzeptoren und/oder mindestens eine Zone einer Siliciumlegierung aufweisenden Submicropartikeln und optional Macropartikeln, insbesondere mit einer Siliciumlegierung beschichteten Silicium Submicropartikeln und optional Clustern der Submicropartikel in Form von Macropartikeln als Siliciumpulver sowie ein Siliciumpulver erhältlich nach dem Verfahren. Erfindungsgemäß umfassend die Submicropartikel eine monokristalline Phase von Silicium und optional zusätzlich mindestens eine Zone umfassend eine Siliciumlegierung. Somit sind die Siliciumpulver vorzugsweise nanokristallin. Folglich sind die erfindungsgemäßen Submicropartikel vorzugsweise einkristallin (single-phase), weiter bevorzugt sind die Submicropartikel einkristallin und weisen eine Partikelgröße von 10 bis 30 nm, bevorzugt um 20 nm +/- 10 nm auf. Besonders bevorzugt sind Siliciumpulver umfassend nanokristalline und monokristalline Submicropartikel.

Besonders bevorzugt sind Submicropartikel ausgewählt aus Submicropartikeln, die
a) im Wesentlichen eine monokristalline Phase einer Siliciumlegierung umfassen, insbesondere eine Silicium und Bor enthaltende Legierung sowie optional mindestens eine Zone einer Silicium und Bor enthaltenden Legierung, und/oder
b) Submicropartikel umfassend dotierte Domänen eine Siliciumlegierung mit kleiner 10²¹ Atomen/cm³, insbesondere kleiner gleich 10²⁰ Atomen/cm³, insbesondere bis 10¹⁸ Atomen/cm ausgewählt aus mindestens einem Elektronendonator und Elektronenakzeptor, wobei die Submicropartikel optional mindestens eine Zone einer Siliciumlegieung umfassen. So können erfindungsgemäße Submicropartikel beispielsweise zumindest teilweise schalenartig von innen nach außen aufgebaut sein und innen im Kern dotierte Domänen und mindestens eine äußere Schale einer Siliciumlegierung umfassen.

Gleichfalls Gegenstand der Erfindung sind Siliciumpulver und ein Verfahren zur Herstellung dieser, indem Siliciumpulver umfassend Submicropartikel erhalten werden, die mindestens ein Submicropartikel umfassen mit einer Siliciumlegierung mit größer gleich 10²¹ Atomen/cm³, insbesondere größer gleich 10²¹ Atomen/cm³ bis 6,2 · 10²² Atomen/cm³, wobei die Atome ausgewählt sind aus mindestens einem Elektronendonator und Elektronenakzeptor. Weiter bevorzugt sind Siliciumlegierungen mit größer gleich 1 Promille, bevorzugt von 1 bis 99% Atomen/cm³ legiert, besonders bevorzug 0,1 bis 10% Atomen/cm³.

Zudem können 0,01 bis 100 Gew.-%, insbesondere größer gleich 20 Gew.-% der Submicropartikel des Siliciumpulvers mindestens eine Zone einer Siliciumlegierung aufweisen, bevorzugt größer gleich 50 Gew.-%.

Gegenstand der Erfindung ist ein Siliciumpulver, wobei das Siliciumpulver Submicropartikel mit einer Partikelgröße von 5 bis 3000 nm, vorzugsweise 10 nm bis 1000 nm aufweist, insbesondere von 20 bis 500 nm, bevorzugt 30 bis 200 nm, besonders bevorzugt von 40 bis 150 nm, weiter bevorzugt von 70 bis 150 nm, und die Partikel im Wesentlichen kristallin sind.
Besonders bevorzugt weisen die Submicropartikel im Wesentlichen eine Partikelgröße von 5 bis 200 nm auf. Dabei ist es weiter bevorzugt, wenn vorzugsweise mindestens ein Submicropartikel lokal eine Siliciumlegierung mit größer gleich 10²¹ Atomen/cm³,(gleichbedeutend bis 6,2 · 10²² Atomen/cm³ im Fall von Bor), ausgewählt aus mindestens einem Elektronendonator und Elektronenakzeptor aufweist.

Alternativ bevorzugt sind Partikelgrößen der Submicropartikel von 5 bis 200 nm, 5 bis 10 nm, 20 bis 200 nm, 40 bis 80 nm, 50 bis 200 nm, um 120 nm bis um 150 nm mit einer Schwankungsbreite von +/- 20 nm. Als im Wesentlichen kristallin oder polykristallin gilt ein Pulver mit einer Kristallinität von größer gleich 15%, insbesondere von größer gleich 16%. Besonders bevorzugt ist eine Kristallinität von größer gleich 85 %, insbesondere größer gleich 90 %, bevorzugt größer gleich 95 % bis 99,9%. Bevorzugte Submicropartikel sind nanokristallin und vorzugsweise einkristallin. Bevorzugt sind auch monokristalline Submicropartikel, insbesondere integral betrachtet, bestehend aus einer Siliciumlegierung. Die Kristallinität kann mittels XRD bestimmt werden (Referenzspektrum: size fraction 97 % kristallin, 3 % amorph (bestimmt durch Einwaage SiO₂ als Referenzspektrum: 97 + 3 %). Alternativ oder zusätzlich kann die Kristallinität mittels XRPD ermittelt werden über die Formel %Kristallinität = (100 x A)/(A + B -C) mit A = gesamte Peakfläche der Reflexe der kristallinen Bestandteilen des Diffraktogramms; B = gesamte Fläche unterhalb der Peakfläche A; C = luftstreuend-, fluoreszierend- und gerätebedingte Untergrundfläche. Gesamte Peakfläche A mit Untergrund genau unterhalb der schmalen Reflexe der Si-Phase. Die Untergrundfläche C wurde anhand der XRD-Diffraktogramm des Si-Referenzstandards NIST 640 (Si-Standard = 100 % Kristallinität) ermittelt. Fläche B entspricht einem eingelegten Untergrundverlauf sowie dem konstanten Untergrund C. Berechnung (HighScore Plus Software). Ein Kristall ist ein anisotroper, homogener Körper, der eine dreidimensional periodische Anordnung der Bausteine besitzt und ein XRPD mit klar definierten auflösbaren Reflexen besitzt.

Nach einer bevorzugten Ausführungsform sind Gegenstand der Erfindung Siliciumpulver erhältlich nach dem erfindungsgemäßen Verfahren, wobei die Siliciumpulver Submicropartikel umfassen und die Submicropartikel eine Partikelgröße von 5 bis 3000 nm aufweisen, insbesondere von 5 nm bis 40 nm, insbesondere von 5 nm bis 20 nm, wobei die Submicropartikel im Wesentlichen kristallin sind und, wobei das Siliciumpulver eine Kristallinität von größer gleich 15,5%, insbesondere von größer gleich 16%. Besonders bevorzugt ist eine Kristallinität von größer gleich 85 % bis kleiner gleich 99,9 % aufweist. Vorzugsweise ist die Kristallinität größer gleich 90 % bis 99,9 %, bevorzugt größer 95 % bis 99,9 %.

Besonders bevorzugte Siliciumpulver umfassen Submicropartikel, die je nach Verfahrensführung definierte Partikelgrößen aufweisen können, wobei besonders bevorzugte Submicropartikelgrößen von 5 bis 200 nm liegen, bevorzugt von 5 bis 150 nm, besonders bevorzugt sind Submicropartikelfraktionen mit Partikelgrößen um 20 nm, 30 nm, 40 nm, 50 nm, 60 nm, 70 nm, 80 nm, 90 nm, 100 nm, alternativ um 40 bis 80 nm, bevorzugt um 30 nm jeweils unabhängig mit einer Abweichung von plus/minus 10 nm, bevorzugt plus/minus 5 nm. Diese Partikel können unmittelbar ohne weitere Wärmebehandlung aus dem Verfahren isoliert werden.

Als besonders bevorzugt gelten Siliciumpulver in denen weniger als 20 % der Submicropartikel eine Abweichung von der mittleren Korngröße d₅₀ und weniger als 5 % eine Abweichung von größer gleich 50 % von der mittleren Korngröße d₅₀ aufweisen.

Bevorzugt sind weiterhin Pulver, deren Submicropartikel einen mittleren Durchmesser d₅₀ (gemessen durch TEM-Auswertung; TEM = Transmissions-Elektronenmikroskop) im Bereich von 5 bis 200 nm, vorzugsweise von 30 bis 70 nm, aufweisen.

Ob die Submicropartikel sich sphärisch oder in der Form von Whiskern ausbilden hängt unter anderem von der H₂-Konzentration bei der Darstellung ab. Je nach Temperaturprofil, Verdünnungsgas (Konzentration, Strömungsgeschwindigkeit) Herstellbedingungen können Submicropartikel isoliert werden oder überwiegend Submicropartikel, die zu Clustern zu Macropartikeln agglomeriert sind, erhalten werden. Als Cluster nach der Erfindung gelten Submicropartikel, die zumindest an einer Stelle miteinander verwachsen sind, wobei ein Submicropartikel an einer Phasengrenze eines anderen Submircopartikels angewachsen sein kann.

Gegenstand der Erfindung sind auch agglomerierte Cluster der Siliciumpulver, die in Form von Macropartikeln vorliegen. Diese Cluster können gebildet werden, indem einzelne kristalline Submicropartikel bei der Herstellung aneinanderwachsen und optional weiterwachsen. Aufgrund der kurzen Kontaktzeiten des erfindungsgemäßen Verfahrens weisen sowohl die isolierten Submicropartikel als auch die in Macropartikeln zu Clustern agglomerierten Submicropartikel im Wesentlichen nur eine kristalline Phase an reinem Silicium auf. Diese Cluster können als lineare Ketten, ähnlich in Form von Drähten oder auch verzweigt vorliegen.

So können bei einer Temperaturführung um 100 bis 500 °C direkt im Anschluss an Zersetzung zur Bildung der Submicropartikel oder im Plasma, vorzugsweise um 500 °C, überwiegend Submicropartikel, insbesondere nicht agglomerierte Submicropartikel, isoliert werden und bei größer 500 °C, insbesondere bei größer gleich 550 °C bis 1300 °C +/- 100°C Cluster von Submicropartikeln als Macropartikeln isoliert werden.

Weiter ist es in einer Alternative bevorzugt, wenn die Submicropartikel in Clustern als Macropartikel vorliegen, wobei die Macropartikel eine Partikelgröße von 10 nm bis 6 µm aufweisen, insbesondere 10 nm bis 3 µm, bevorzugt 400 nm bis 6 µm, besonders bevorzugt 100 nm bis 3 µm, vorzugsweise 300 nm bis 3 µm, 500 nm bis 3 µm, 1 bis 3 µm.

Ihre Herstellung erfolgt bevorzugt um 100 °C sowie bei bis zu 700°C +/- 100 °C im kalten Plasma. Das kalte Plasma wird mittels transienter Hochspannungsentladung in einer bipolaren Elektrodenanordnung (Nullpotential, Hochspannungselektrode), die additiv mit einer dielektrischen Barriere ausgestattet sein kann, erzeugt. Die bevorzugten Betriebsparameter für eine planare Elektrodenanordung mit einer Gasschlagweite (GAP) von rund 5 mm sind 15 kVp am Gasraum anliegendes Potential mit einer Impulsdauer von gerundet 950 Nanosekunden und einer Wiederholrate von gerundet 8 000 pro Sekunde. Ein typischer spezifischer Prozessgasflow mit 1 % Monosilan in Argon Matrix liegt gerundet bei 40 cm/s gleichbedeutend mit einem Volumenflow von 2400 cm³/min geteilt durch 1 cm² Elektrodenfläche.

Gegenstand der Erfindung sind auch Siliciumpulver umfassend Submicropartikel mit im Wesentlichen einer monokristallinen, reinen Silicium-Phase oder einer Siliciumlegierung sowie optional zusätzlich mindestens eine Zone einer Silicium und Bor enthaltenden Legierung.

Ebenfalls Gegenstand der Erfindung sind nach einer Ausführungsvariante Siliciumpulver, insbesondere umfassend Submicropartikel und/oder Macropartikel, a) die jeweils unabhängig aus einer Siliciumlegierung bestehen und/oder, b) die jeweils unabhängig mindestens eine intrinsische Zone einer Siliciumlegierung mit mindestens einem Elektronendonator oder mindestens einem Elektronenakzeptor mindestens teilweise aufweisen und/oder, c) jeweils unabhängig mindestens eine Zone einer Siliciumlegierung umfassend einen Elektronendonator, Elektronenakzeptor, eine einen Elektronendonator oder Elektronenakzeptor enthaltenden Verbindung als eine mindestens teilweise Beschichtung aufweisen, insbesondere liegt die mindestens eine Zone aus äußere Beschichtung auf den Submicropartikeln und optional Macropartikeln vor, vorzugsweise sind die Elektronendonatoren und -akzeptoren ausgewählt aus einem Element der Gruppe 13 oder 15 des Periodensystems (PSE) der Elemente nach IUPAC, bevorzugt Bor, Aluminium, Phosphor, Arsen und/oder Antimon. Insbesondere sind sie mit einer Zone der Siliciumlegierung teilweise beschichtet, vorzugsweise im Wesentlichen vollständig beschichtet.

Ferner sind Gegenstand der Erfindung Siliciumpulver die eine Siliciumlegierung mit einem Element der Gruppe 13 oder 15 des Periodensystems (PSE) der Elemente nach IUPAC aufweisen und/oder zumindest eine Zone einer Siliciumlegierung mit einem Element der Gruppe 13 oder 15 aufweisen.

Gegenstand der Erfindung sind auch Siliciumpulver, deren a) Submicropartikel aus einer Siliciumborlegierung bestehen, und/oder b) deren Submicropartikel mindestens eine Zone einer Siliciumborlegierung umfassen, insbesondere liegt die Zone als eine zumindest teilweise Beschichtung der Submicropartikel vor. Alternativ können auch mehrere Zonen oder mehrere Beschichtungen auf die Submircopartikel und/oder Macropartikel aufgebracht sein.

Domänen sind nach der Erfindung dotierte Bereiche der Siliciumpartikel, während die Zonen Bereich von Siliciumlegierungen umfassen.

Ferner werden nach einer Alternative Siliciumpulver beansprucht, deren Submicropartikel Bor enthalten, indem sie eine zumindest teilweise bis überwiegend Bor enthaltende Zone aufweisen, und optional können die Submicropartikel dotierte Domänen aufweisen und/oder eine vollständige Bor enthaltende Zone aufweisen. Alternativ oder zusätzlich können die Partikel in der Matrix dotiert sein.

Beispielswiese können Bereich der Submicropartikel und/oder Macropartikel mindestens eine Zone einer Siliciumlegierung aufweisen, wie eine innenliegende Siliciumlegierungszone und außen können die Siliciumpartikel zusätzlich zumindest teilweise mit Elektronendonatoren oder - aktzeptoren dotiert sein.

Zonen umfassen definierte dreidimensionale Bereiche in den Submicropartikeln, bevorzugt sind Zonen, die dem Kern des Partikels entsprechen und mindestens ein äußerer Bereich des Partikels entspricht einer weiteren äußeren Zone einer abweichenden Zusammensetzung. Der Kern des Submicropartikels kann bis zu 95 Volumen-%, insbesondere bis zu 99,5 Vol.-% des Gesamtsubmicropartikels entsprechen. Die äußere Zone entspricht dann 0,5 bis 5 Vol.-% des Gesamtpartikels des Submicropartikels. Eine zusätzliche Zone kann als mindestens teilweise Beschichtung auf den Macropartikeln vorliegen.

Entsprechend einer Alternative umfassen die Siliciumpulver vorzugsweise Submicropartikel, die im Wesentlichen polyedrischen Habitus aufweisen, insbesondere oktaedrisch. Entsprechend einer weiteren bevorzugten Ausführungsform sind die Submicropartikel der Siliciumpulver im Wesentlichen nicht porös und weisen daher im Wesentlichen keine innere Oberfläche auf.

Um die elektrischen Eigenschaften der Siliciumpulver auf die jeweilige Anforderung einstellen zu können, ist es bevorzugt, wenn die Partikel eine zumindest teilweise oder vollständige Bor enthaltende Zonen aufweisen. Die Schichtdicke der Zone kann 1 nm bis 100 nm, insbesondere 1 bis 20 nm betragen, besonders bevorzugt sind 1 bis 5 und 3 bis 10 nm. Die Bestimmung kann mittels hochauflösendem TEM erfolgen.

Eine einfache Bestimmung solcher Zonen kann qualitativ aus den Grauwerten der hochauflösenden ATEM-Bilder erfolgen. Integral kann dies über die dem Fachmann geläufige Bestimmung der Ladungsträgerdichte und Beweglichkeit aus der Seebeck-Gleichung erfolgen.

Für die Bestimmung des Seebeck Koeffizient und des Elektrischer Widerstands im Temperaturbereich -100 bis 500°C und RT bis 1500°C wird das Gerät der Firma Linseis LSR-3 verwendet. Aus dem Seebeckkoeffizenten kann die Ladungsträgerdichte durch lineare Differenzation gewonnen werden. Mit Hilfe der dem Fachmann bekannten Umrechnung ergibt sich aus Ladungsträgerdichte (cm⁻³) dividiert durch das Integral der Ladungsträger (V*cm/s) die Ladungsträgerbeweglichkeit (cm²/V/s).

Bevorzugte Siliciumpulver umfassen Partikel, insbesondere Submicropartikel und/oder Macropartikel, von größer gleich 99, Gew.-% Silicium bevorzugt mit einem Gehalt von größer gleich 99,99 Gew.-%, besonders bevorzugt mit einem Gehalt von größer gleich 99,9999 Gew.-%. Alternativ können die Silicium-Partikel im Wesentlichen ohne äußere Matrix, ohne Siliciumlegierung oder ohne Zone vorliegen, die auch eine passivierende Zone, wie Bor, Oxidschicht umfassen kann. Nach einer Alternative liegen die Silicium-Partikel mit einer Siliciumdioxid-Zone (Core-shell) von typisch 1 nm vor. Ferner umfassen die Siliciumpulver Submicropartikel und optional Macropartikel entsprechend mit Siliciumlegierungen von Bor, Phosphor, Antimon, Arsen und/oder Kohlenstoff etc., insbesondere entsprechende Siliciumlegierungen mit Bor oder Phosphor bzw. Antimon.

In den erfindungsgemäßen Siliciumpulvern weisen die Submicropartikel und optional die Macropartikel umfassend die Submicropartikel in der Gesamtzusammensetzung einen Gehalt von kleiner gleich 2 % Atome/cm³, vorzugsweise kleiner gleich 2000 ppm-Gew Sauerstoff auf, vorzugweise kleiner gleich 1000 ppm-Gew , insbesondere kleiner 10 ppm-Gew., bevorzugt kleiner gleich 1 ppb-Gew bspw. bis zur Nachweisgrenze.

Der Gehalt an Elementen wie Xenon, Argon, Krypton oder auch Stickstoff, die aus dem Herstellungsprozess herrühren, beträgt in der Gesamtzusammensetzung Silicumpulver umfassend Submircropartikel und optional Macropartikel kleiner 5 % Atome/cm³, insbesondere kleiner gleich 10000 ppm-Gew., bevorzugt um 100 ppm-Gew., besonders bevorzugt 1 ppb-Gew bis zur Nachweisgrenze, bspw. bis 1 ppt-Gew. Diese Elemente können die Zahl der dem Fachmann bekannten Gitterfehlstellen beeinflussen, was sich in einer höheren Ladungsträgerbeweglichkeit nach außen darstellt.

Die Analyse erfolgt neben ICP-MS und HP-GD-MS (High Purity Glow Discharge Mass Spectroskopy) bevorzugt mittels Neutronenaktivierungsanalyse (NAA). Die NAA ist eine hochempfindliche physikalische Technik der analytischen Chemie zur qualitativen und quantitativen Spurenanalyse, bei der die zu untersuchende Probe mit Neutronen (oder anderen Teilchen) beschossen wird.

Bei der NAA wird eine Probe von nur wenigen Milligramm (oder Fallweise wenige µg) dem Neutronenstrom (eines Kernreaktors) ausgesetzt. Die Neutronen reagieren mit den Kernen der Probe und machen aus den stabilen Isotopen radioaktive Isotope, deren Massenzahl eins höher ist als die Massenzahl des stabilen Isotops. Bei diesem ersten Kernprozess wird ein promptes γ - Quant ausgesandt, dessen Energie man messen kann und das Auskunft über den Ausgangskern gibt. In den meisten Untersuchungen wird aber erst der Zerfall des entstandenen radioaktiven Kerns zur Analyse verwendet. Es zerfällt anschließend mit der für ihn typischen Halbwertzeit unter Aussendung eines Betateilchens und charakteristischer Gammastrahlung , die in einem Gammaspektrometer untersucht wird. Auf diese Weise sind praktisch alle vorkommenden Elemente in einer Probe quantitativ und qualitativ nachweisbar. Aus den bekannte Eigenschaften der Atomkerne lassen sich neben den Gehalt der Elemente, sogar ihrer Isotope bestimmen. Die Messungen können bspw. am Berlin Neutron Scattering Center durchgeführt werden.

Die Nachweisgrenzen für die Bestimmung von Xenon (Xe), Argon (Ar), Krypton (Kr), oder auch Stickstoff durch Neutronenaktivierung bei einer Bestrahlzeit von 1 Stunde bei einer sogenannten Flußdichte an thermischen Neutronen von 10¹⁴ cm⁻² s⁻¹ liegen bei ca. 10⁻⁸ g (Ne, Xe), ca. 10⁻⁹ (Kr), ca. 10⁻¹¹ (Ar) und ca. 10⁻⁵ g für Sauerstoff (O₂). Die Nachweisgrenze kann damit insbesondere für kleine Probenmengen kleiner gleich 1000 ppm-Gew., um 10 ppm-Gew., 1 ppb-Gew bzw. bis zur Nachweisgrenze, bspw. bis 1 ppt-Gew. betragen bspw. bis 1 ppt-Gew.

Gleichfalls bevorzugt sind Gemische von Siliciumpulvern a) mit hohem Gehalt an Siliciumlegierungen von größer gleich 5 Gew.-% bis 100 Gew.-%, insbesondere 10 bis 50 Gew.-%, mit b) Siliciumlegierungen mit einem geringen Gehalt an Siliciumlegierung von kleiner gleich 4 Gew.-% bis 1 ppt.-Gew.%, vorzugsweise kleiner gleich 2 Gew.-% bis 10 ppm.-Gew.%, und optional Siliciumpulvern c) die keine Siliciumlegierung umfassen und optional dotiert mit den genannten Dotiermitteln dotiert sein können. Das Verhältnis der Siliciumpulver a) zu b) zu c) kann für a : b : c für a 1 bis 1000 für b 1 bis 1000 und für c 0 bis 1000 betragen.

Das reine Siliciumpulver mit einem Gehalt an Silicium von größer gleich 95 Gew.-% kann ein Legierungsprofil aufweisen an Bor, Phosphor, Arsen, Aluminium, Eisen, Natrium, Kalium, Nickel, Chrom, Schwefel, Barium, Zirkon, Zink, Titan, Calcium, Magnesium, Kupfer, Chrom, Cobalt, Zink, Vanadium, Mangan und/oder Blei von unter 2% bis 1 ppb, bevorzugt von 2 % und 0,1 % (Atome/cm³) in Bezug auf die Gesamtzusammensetzung an Siliciumpulver.

Zur Herstellung der hochreinen, d.h. größer 95% Silicium oder reinen Siliciumpulver wird ein reines bis hochreines Silan eingesetzt, wobei die Definition von rein bis höchstreines Silan mit Halbleiterqualität verwendet werden, wie monomeres und/oder polymeres Monosilan, H-Silan und/oder teilhalogenierte, bis perhalogenierte Silane, wie Chlorsilan, insbesondere der allgemeinen Formel I, II und/oder III oder eine Mischung dieser, wie höchstreines Tetrachlorsilan, höchstreines Trichlorsilan und/oder höchstreines Dichlorsilan, vorzugsweise mit einem Gehalt an einem Silan von 80 bis 99,9999999 Gew.-%, ad 100 Gew.-% ggf. Polysilane. Alternativ kann statt einem einzelnen Silan auch eine Mischung von Silanen eingesetzt werden, sofern sie dem vorgenannten Anforderungsprofil an den Gehalt des Silans entspricht.

Alternativ können die Siliciumpulver Submicropartikel und optional Cluster der Submicropartikel umfassen, die mit Kohlenstoff versehen und/oder zumindest teilweise beschichtet sind.

Gegenstand der Erfindung sind auch Siliciumpulver umfassend Submicropartikel mit im Wesentlichen einer monokristallinen Phase einer Siliciumlegierung, insbesondere eine Silicium und Bor enthaltenden Legierung sowie optional mindestens eine Zone einer Silicium und Bor enthaltenden Legierung. Bor und Phosphor stehen hier stellvertretend für weitere, dem Fachmann bekannte Legierungselemente. Ebenso Gegenstand der Erfindung sind Siliciumpulver umfassend Submicropartikel mit im Wesentlichen einer monokristallinen Phase oder Domäne von dotiertem Silicium, insbesondere einer Silicium und Phosphor oder Silicium und Bor enthaltenden Phase sowie optional mindestens eine Zone einer Silicium und Bor oder Phosphor enthaltenden Legierung. Bor und Phosphor sind stellvertretend für weitere, dem Fachmann bekannte Legierungselemente, genannt.

Ebenso Gegenstand der Erfindung sind Siliciumpulver, die Submicropartikel und optional Cluster der Submicropartikel in Form von Macropartikeln umfassen, die mit mindestens einem Elektronendonator (n-dotierte Halbleiter) und/oder mindestens einem Elektronenakzeptor (p-Halbleiter) zusätzlich in einer Domäne dotiert sind. Besonders bevorzugt umfassen die legierten Siliciumpulver Submicropartikel und/oder Cluster, die zusätzlich mindestens eine Domäne mit einer Kombination einer p-dotierten und eine mindestens eine Domäne mit einem n-dotierten Silicium aufweisen, insbesondere weisen die Partikel und/oder Cluster mindestens zwei Domänen von p- und n-dotiertem Silicium auf. Besonders bevorzugt sind Siliciumpulver umfassend Partikel und/oder Cluster als mindestens ein Halbleiter, der mindestens einen p-n-Übergang, p-p- und/oder n-n-Übergang aufweist. Bevorzugte Metalle zur Bildung der dotierten Domänen umfassen Phosphor, Arsen, Antimon, Wismut, Bor, Aluminium, Gallium, Indium, Thallium, Europium, Erbium, Cer, Praseodym, Neodym, Samarium, Gadolinium, Terbium, Dysprosium, Holmium, Thulium, Ytterbium, Lutetium, Lithium, Germanium, Eisen, Ruthenium, Osmium, Kobalt, Rhodium, Iridium, Nickel, Palladium, Platin, Kupfer, Silber, Gold, Zink, oder aus einem Gemisch dieser.

Entsprechend einer besonders bevorzugten Ausführungsform weist das Siliciumpulver, insbesondere die Submicropartikel und optional die Macropartikel des Siliciumpulvers, thermoelektrische Eigenschaften auf. So können diese Siliciumpulver mit thermoelektrischen Eigenschaften vorzugsweise in einem thermoelektrischen Generator verwendet werden. Zur Verbesserung der thermoelektrischen Eigenschaften der Siliciumpulver können in das Kristallgitter der kristallinen Submicropartikel Schweratome eingebaut werden. Über den gezielten Einbau von Streuzentren kann der Wärmetransport in den Siliciumpulvern reduziert werden. Bevorzugte Metalle als Schweratome umfassen Wismut, Gallium, Indium, Thallium, Europium, Erbium, Cer, Praseodym, Neodym, Samarium, Gadolinium, Terbium, Dysprosium, Holmium, Thulium, Ytterbium, Lutetium, Eisen, Ruthenium, Osmium, Kobalt, Rhodium, Iridium.

Diese Macropartikel weisen eine moderate Oberfläche und starke Verzweigung auf, so dass der Wärmetransport behindert wird. Eine niedrige thermische Leitfähigkeit bei hoher elektrischer Leitfähigkeit, zieht wiederum eine hohen Seebeck Koeffizient alpha (in µV/K) nach sich.

So führt dargestelltes Material, deren elektrische Leitfähigkeit auf 800 S●·cm⁻¹ bei 200 °C und deren Wäremleitfähigkeit auf rund 5 W·m⁻¹ ● K⁻¹ bei 200 °C bestimmt wurden zu einem Seebeck-Koeffizienten von rund 300 Miko-V ●·K⁻¹, aus dem wiederum ein z.T. (als sogenannter thermoelektrischer Performaceparameter) von rund 0,45 bei 200 °C bestimmt wurde.

Entsprechend einer vorzugsweisen Ausführungsform ist das Siliciumpulver ein Verbindungshalbleiter. Übliche Thermoelemente aus Metallen wandeln thermische Energie nur sehr ineffizient in elektrische Energie um. Mit den erfindungsgemäßen Partikeln mit thermoelektrischen Eigenschaften können auf einfache und wirtschaftliche Weise thermoelektrische Partikel in einem großtechnischen Verfahren hergestellt werden. Eine wirtschaftliche Funktionszone (Zone), die mit heißen Komponenten, Oberflächen, heißen Dämpfen oder Gasen in Kontakt kommen, kann aus erfindungsgemäßen Partikeln hergestellt werden. Auch Festkörper können daraus hergestellt werden. Somit gelingt es mit Hilfe der thermoelektrischen Partikel aus Abwärme elektrische Energie zu gewinnen. Besonders bevorzugte Anwendungsfelder bieten Verbrennungsmotoren und die mit den Abgasen in Kontakt kommenden Vorrichtungen, insbesondere deren Oberflächen, Gas- oder Dampfturbinen sowie die damit verbauten Vorrichtungen, Flugzeugturbinen oder auch Batterien, die große Hitze entwickeln.

Ebenso Gegenstand der Erfindung ist ein Verfahren zur Herstellung von Siliciumpulver sowie Siliciumpulver erhältlich nach diesem Verfahren, indem
a) mindestens eine bei erhöhter Temperatur gasförmige Siliciumverbindung, nach einer Alternative optional in Gegenwart eines bei erhöhter Temperatur reaktiven Gases, und
b) optional in Gegenwart eines Verdünnungsgases, insbesondere eines unter den Verfahrensbedingungen inerten Verdünnungsgases, in einer im Wesentlichen sauerstofffreien Atmosphäre unter (i) thermischen Bedingungen und/oder (ii) im Plasma zersetzt wird, und in Form eines Siliciumpulvers erhalten wird umfassend Submicropartikel deren Partikelgröße bei 5 bis 3000 nm liegt, insbesondere von 10 nm bis 300 nm, vorzugsweise von 10 bis 150 nm, insbesondere werden die Siliciumpulver abgeschieden, wobei die Submicropartikel, im Wesentlichen kristallin sind.

Besonders bevorzugt ist ein Verfahren in dem Siliciumpulver mit mindestens einen Submicropartikel umfassend eine Siliciumlegierung mit größer gleich 10²¹ Atomen/cm³ ausgewählt aus mindestens einem Elektronendonator und/oder Elektronenakzeptor hergestellt werden können. Bevorzugt kann eine definierte Vielzahl an Submicropartikeln mit einer Siliciumlegierung hergestellt werden. Zusätzlich zu den genannten Siliciumlegierungen können Domänen vorliegen, in denen das Silicium dotiert ist.

Ferner ist Gegenstand der Erfindung ein Verfahren in dem mindestens teilweise die Submicropartikel in Clustern als Macropartikel erhalten werden.

Gegenstand der Erfindung ist auch ein Verfahren zur Herstellung der Siliciumpulver umfassend mindestens den Schritt Zersetzung (A) der Siliciumverbindung, optional im Gemisch mit einem Verdünnungsgas und optional mindestens eines reaktiven Gases, unter (i) thermischen Bedingungen und/oder (ii) im Plasma, und Ausbildung von Submicropartikeln und/oder Clustern der Submicropartikel in Form von Macropartikeln innerhalb von 1 bis 10000 Millisekunden, vorzugsweise von 10 bis 4000 Millisekunden. Weiter bevorzugte alternative Zeiten umfassen nach einer Alternative innerhalb von 10 bis 100 Millisekunden, vorzugsweise innerhalb von 20 bis 80 Millisekunden, bevorzugt um 60 Millisekunden, vorzugsweise mit plus/minus 5 Millisekunden, und nach einer Alternative innerhalb von 50 bis 1000 Millisekunden, insbesondere innerhalb von 100 bis 500 Millisekunden erfolgt, bevorzugt kleiner gleich 100 bis 400 Millisekunden. Dabei ist es besonders bevorzugt, wenn die Temperatur von 1000 bis 1700 °C, vorzugsweise von 1100 bis 1600 °C mit einer Schwankung von plus/minus 50 °C beträgt. Die vorstehenden Angaben in Millisekunden entsprechen vorzugsweise der Verweilzeit/ Zeitspanne im Reaktionsraum, besonders bevorzugt im Raum der Erzeugung chemischer Radikale und der daraus sich ergebende Partikelbildung. Nach einer Verfahrensalternative kann die Verweilzeit auch die Zeit zur Bildung der Siliciumlegierung und/oder der mindestens einen Zone einer Siliciumlegierung mitumfassen und nach einer anderen Alternative entspricht die Verweilzeit der Zeitspanne ohne Bildung der Siliciumlegierung und/oder Zone. Alternativ kann zu einem definierten Zeitpunkt die Bildung einer Zone einer Siliciumlegierung der Submicropartikel und optional Macropartikel durch die Gegenwart einer Silicium-Verbindung und eines reaktiven Gases erfolgen, dessen Zersetzungsprodukte sich auf den Submicropartikeln und optional Clustern als Zone abscheiden.

Vorzugsweise erfolgt der Schritt der Zersetzung, (A) der Siliciumverbindung optional in Gegenwart des Verdünnungsgases unter (i) thermischen Bedingungen und/oder (ii) im Plasma, und Ausbildung von Submicropartikeln und/oder Macropartikel in Form von Clustern der Submicropartikel innerhalb von 10 bis 4000 Millisekunden, insbesondere von 100 bis 4000 Millisekunden. Des Weiteren ist es bevorzugt, wenn a) eine gasförmige Siliciumverbindung und optional in Gegenwart eines Verdünnungsgases unter (i) thermischen Bedingungen bei einer Temperatur von 800 bis 1800 °C zersetzt wird.

Bevorzugt erfolgt die thermische Zersetzung mit den Schritten a) eine gasförmige Siliciumverbindung wird optional b) in Gegenwart mindestens eines Verdünnungsgases unter (i) thermischen Bedingungen bei einer Temperatur von 800 bis 1800 °C zersetzt, insbesondere von 1000 bis 1700 °C, vorzugsweise um 1300 °C bis 1600 °C. Besonders bevorzugt ist eine Zersetzung bei Temperaturen um 1300 bis 1800 °C, insbesondere innerhalb von 60 bis 800 Millisekunden, bevorzugt innerhalb von 200 bis 800 Millisekunden, besonders bevorzugt innerhalb von 300 bis 600 Millisekunden, vorzugsweise um 400 Millisekunden. Die Abkühlung zur Abscheidung der Siliciumpulver umfassend die Submicropartikel erfolgt vorzugsweise unmittelbar anschließend auf eine Temperatur unter 1300 °C, vorzugsweise unter 1000 °C, bevorzugt auf unter 600 °C innerhalb eines Zeitraumes von größer gleich 60 Millisekunden, insbesondere größer gleich 80 Millisekunden, bevorzugt innerhalb größer gleich 100 Millisekunden.

Erfindungsgemäß wird hochreines Monosilan optional in Gegenwart eines Verdünnungsgases unter thermischen Bedingungen zersetzt. Vorteilhaft kann auch eine Gasphasenabscheidung von Methylsilan bei 1250 bis 1600 °C erfolgen, bevorzugt um 1300 bis 1600 °C, insbesondere mit einer Abkühlung auf unter 1000 °C in einem Zeitraum größer gleich 100 Millisekunden.

Besonders bevorzugt ist eine Verfahrensalternative, in der mindestens eine Siliciumverbindung und optional in Gegenwart mindestens eines Verdünnungsgases unter (i) thermischen Bedingungen bei einer Temperatur von 800 bis 1800 °C zersetzt wird, vorzugsweise von 1300 bis 1700 °C, besonders bevorzugt von 1400 °C bis kleiner gleich 1600 °C plus/minus 50 °C, wobei überwiegend kristalline Silicium Submicropartikel und optional Cluster der kristallinen Submicropartikel isoliert werden können. Ein bevorzugtes im Verfahren durchlaufenes Temperaturprofil umfasst Temperaturen größer gleich 1000 °C bis kleiner gleich 1800 °C und anschließend kleiner gleich 900 °C. Wobei die Temperatur um 50 °C schwanken kann. Besonders bevorzugt erfolgt die Zersetzung in einem Hotwall-Reaktor und die Abscheidung in einer nachgeschalteten Filteranordung.

Die Bildung der Siliciumpulver kann erfolgen durch 1) Zersetzen der Siliciumverbindung, 2) Abkühlen des gasförmigen Siliciums, insbesondere in Gegenwart mindestens eines inerten Verdünnungsgases und/oder 3) die Siliciumpulver gebildet werden durch ein Inkontaktbringen der zersetzten Siliciumverbindung mit einem kühleren reaktiven Gas unter Bildung und Abkühlen der beschichten Siliciumpulver und Abscheidung des Pulvers.

Eine alternative Verfahrensführung umfasst die Zersetzung (A) der mindestens einen Siliciumverbindung in Gegenwart eines Verdünnungsgases und mindestens reaktiv Gases oder einer Mischung von reaktiven Gasen unter (i) thermischen Bedingungen und/oder (ii) im Plasma, und Ausbildung von Submicropartikeln umfassend eine Siliciumlegierung und/oder Macropartikeln in Form von Clustern der Submicropartikel, und optional Ausbildung einer Zone einer Siliciumlegierung, mit den Schritten
- Ausbildung der Siliciumlegierung (B) und Bildung der Submicropartikel und/oder Cluster der Submicropartikel unter (i) thermischen Bedingungen und/oder in (ii) einer Behandlung im Plasma und/oder
- Ausbildung mindestens einer Zone einer Siliciumlegierung (C) auf gebildeten Submicropartikeln und/oder Clustern der Submicropartikeln, unter (i) thermischen Bedingungen und/oder in (ii) einer Behandlung im Plasma. In einer Alternative werden die Submicropartikel aus nanonkristallinem Silicium gebildet und mit einer Zone einer Siliciumlegierung zumindest teilweise beschichtet und/oder mit Siliciumcarbid beschichtet. In einer weiteren Alternative werden die Macropartikel entsprechend beschichtet.

Ferner ist Gegenstand der Erfindung ein Verfahren umfassend vorzugsweise die Schritte (i) thermische Zersetzung zumindest teilweise an einer heißen, insbesondere von der heißen Innenwand beabstandeten, Innenzone in einem Reaktionsraum und/oder (ii) im Plasma in einem Reaktionsraum in einem Reaktor, insbesondere einem Free-Space Reaktor, umfassend einen Reaktionszylinder, über mindestens eine Zuführung die Siliciumverbindung, insbesondere in dem im Bereich des Reaktoranfangs in den Reaktionsraum eines Reaktionszylinders, wobei die Siliciumverbindung, optional als Gemisch umfassend das reaktive Gas und/oder das Verdünnungsgas in den Reaktionsraum eingebracht wird. Der Reaktionsraum umfasst mindestens eine Reaktionszone, vorzugsweise umfasst der Reaktionsraum eine Vielzahl an definierten Reaktionszonen, in denen die Temperatur definiert einstellbar ist. In mindestens einer Reaktionszone erfolgt die Zersetzung der Siliciumverbindung und optional die Bildung der Siliciumpulver, und optional erfolgt die Bildung der Submicropartikel umfassend die Siliciumlegierung und/oder die Bildung der Zone der Siliciumlegierung der gebildeten Siliciumpulver, am Reaktorausgang werden die gebildeten Submicropartikel und/oder Macropartikel in Form von Clustern der Submicropartikel erhalten.

Dabei ist es bevorzugt, dass die Submicropartikel und/oder Cluster der Submicropartikel in einer Vorrichtung zur Partikelabscheidung aufgefangen werden, insbesondere mittels einer gasdurchlässigen Membran alternativ mit einem Filterschlauch.

Verfahrensgemäß kann die Bildung der Siliciumlegierung der Submicropartikel und optional der Submicropartikel der Cluster erfolgen mit a) Elementen der dritten Hauptgruppe des Periodensystems der Elemente (Gruppe 13 PSE IUPAC), wie Bor, Aluminium, und/oder Elementen der fünften Hauptgruppe (Gruppe 15 PSE IUPAC), wie Stickstoff, Phosphor, Arsen, Antimon, bevorzugt kann eine Legierungsbildung durch Zusatz von Diboran erfolgen, und/oder es kann eine Modifizierung der Submicropartikel und optional der Cluster erfolgen b) zumindest teilweise mit mindestens einem organofunktionellen Silan. In dem Verfahren werden vorzugsweise Verbindungen der vorgenannten Elemente (reaktive Gase) der dritten Hauptgruppe des Periodensystems der Elemente (Gruppe 13), wie Bor, Aluminium, und/oder Elementen der fünften Hauptgruppe (Gruppe 15 PSE IUPAC), Phosphor, Arsen, Antimon zersetzt.

Besonders bevorzugte Abkühlungsbedingungen werden nachfolgend näher erläutert. Wie vorstehend ausgeführt kann die Bildung der Pulver auf zweierlei Weise erfolgen, so kann die Abkühlung und Ausbildung der Siliciumpulver durch Quenchen mit kühlen reaktiven Gasen, insbesondere flüssigem Stickstoff oder einem anderen sich hier inert verhaltenem Gas/Element oder Verbindung erfolgen ggf. mit einem Gehalt an verdampfenden Kohlenwasserstoffen, Bor-, Phosphor-Verbindungen und/oder Verbindungen der Elemente der dritten Hauptgruppe des Periodensystems der Elemente (Gruppe 13 PSE IUPAC), wie Bor, Aluminium, und/oder Elemente der fünften Hauptgruppe (Gruppe 15 PSE IUPAC), wie Stickstoff, Phosphor, Arsen, Antimon.

Die Abkühlung der Siliciumpulver kann neben dem physikalischen Quenchen im Millimeter, bevorzugt im Mikrometerspalt zweier rotierender Metallwalzen auch erfolgen in einer kühleren Region des Reaktors, die vorzugsweise eine Temperatur im Bereich kleiner gleich 1000 °C aufweist. Eine Abkühlung kann auch durch ein Einleiten in inerte, kühle und leicht verdampfbare Flüssigkeiten oder durch Einleiten in flüssige Siliciumverbindungen oder Bor enthaltende Verbindung erfolgen. Generell kann das gasförmige Silicium abgekühlt werden, indem es in eine Zone des Reaktors oder Reaktionszone geleitet wird, dessen Temperatur deutlich unterhalb 1300 °C liegt, vorzugsweise unter 1000 °C, weiter bevorzugt unterhalb 900 °C, besonders bevorzugt unterhalb 800 °C. Besonders bevorzugt erfolgt ein rasches Abkühlen durch Einstellen einer Temperaturdifferenz von größer gleich 50 °C, insbesondere größer gleich 500 °C, bevorzugt von größer gleich 200 °C, besonders bevorzugt größer gleich 100 °C unterhalb des Schmelzpunktes von Silicium oder des Schmelzpunktes der Siliciumlegierung, innerhalb von größer gleich 100 Millisekunden, um im Wesentlichen kristallines Siliciumpulver zu erhalten. Vorzugsweise wir im Verfahren auf kleiner gleich 100 °C abgeschreckt, insbesondere auf 10 bis 60 °C.

Um im Wesentlichen kristalline Siliciumpulver zu erhalten, erfolgt vorzugsweise eine definierte Abkühlung innerhalb einer größeren Zeitspanne indem eine kontinuierliche definierte Temperaturerniedrigung erfolgt. So kann die Zone zur Zersetzung und/oder Legierungsbildung eine Temperatur von 800 bis 2500 °C aufweisen, während die Abscheidung und optional Bildung einer Siliciumlegierung bei - 298 °C bis kleiner gleich 1300 °C, vorzugsweise unter 900 °C erfolgt.

Besonders effizient zur Herstellung kristalliner, beschichteter Partikel ist eine Verfahrensführung analog der Sprühtrocknung, in dem mit einem starken Druckabfall von bspw. 1 bar auf 200 mbar(abs) gearbeitet wird, die kristallinen Siliciumpartikel optional in Gegenwart eines Verdünnungsgases mit einem reaktiven Gas zur Reaktion gebracht werden, um in der Zone einer Siliciumlegierung auf die Partikel und optional auf die Cluster aufzubringen.

Alternativ können die kristallinen Submicropartikel erhalten werden in einem nicht-thermische Plasma, dessen Temperaturen bei kleiner gleich 1300 °C, vorzugsweise kleiner gleich 500 °C liegen. Erfindungsgemäß bevorzugt umfasst das Plasma die Bedingungen einer Gasentladung, insbesondere im nicht-thermischen Gleichgewicht mit einer nachgeschalteten Verweilzeitstrecke bei hoher Temperatur als Temperstrecke. Das Partikelwachstum erfolgt als Kondensation aus einer übersättigten Phase durch Zersetzung des entsprechenden Precursors im Plasma.

Erfindungsgemäß eingesetzte nicht-thermische Plasmen werden beispielsweise durch eine Gasentladung oder durch Einstrahlung von elektromagnetischer Energie, wie durch Einstrahlung von Radio- oder Mikrowellen, in einer Unterdruckkammer erzeugt. Die Erzeugung des Plasmas erfolgt also nicht wie bei thermischen Plasmen durch hohe Temperaturen, sondern durch nicht-thermische Ionisationsprozesse. Dem Fachmann sind solche Plasmen bekannt.

Für die vorstehend aufgeführten Verfahren wurden im nichtthermischen Gleichgewicht betriebene Gasentladungen verwendet. Nichtthermisch bedeutet im erfinderischen Sinne, dass die Elektronen als energievermittelnde Spezies eine höhere Temperatur bzw. eine höhere Energie als die Schwerteilchen von 10¹⁴ cm⁻² s⁻¹ liegen bei ca. 10⁻⁸ g (Ne, Xe), ca. 10⁻⁹ (Kr), ca. 10⁻¹⁰ (Ar) und bei ca. 10⁻⁵ g für Sauerstoff (O₂) aufweisen. Diese wurden durch dem Fachmann bekannte bzw. geläufige Netzteile erzeugt. Das nicht-thermische Plasma weist im Allgemeinen Elektronen mit einer Energie im Bereich von 0,1 bis 100 eV, insbesondere von 1 bis 50 eV, auf und Schwerteilchen mit einer Energie im Bereich von 1 bis 10 eV, insbesondere 0,5 bis 1 eV. Elektrodenabstand 4 mm.

Überraschenderweise hat sich gezeigt, dass sich eine solche nicht-thermische Gasentladung vorteilhaft durch Dimmen über die Pulsfrequenz erzeugen lässt, wobei vorteilhafterweise die Elektroden als Hohlelektroden mit vorzugsweise porösen Stirnflächen bspw. aus Sintermetall durch eine zweidimensionale parabolische Form ausgeführt sind. Damit verteilt sich der 15 Gasstrom gleichmäßig über die Elektrodenoberfläche. Die zweidimensionale pilzartige Oberfläche kann gemäß F(r) = r2 (0,1 < r < 1,1cm) beschrieben werden. Das erfindungsgemäße Verfahren wird im Allgemeinen in nicht-thermischen Plasmen durchgeführt, welche Schwerteilchen-Temperaturen von 50 bis 1400 °C, vorzugsweise von 100 bis 1000 °C aufweisen.

Das Plasma kann gepulst werden, vorzugsweise wird ein im Wesentlichen zylinderförmiges Plasma, insbesondere nicht-thermisches Plasma, in einem zylindrischen Bereich des Reaktionszylinders vorgesehen, in dem eine vorteilhaft homogene Dotierung und/oder Zone einer Siliciumlegierung mit Zersetzungsprodukten aus einem reaktiven Gas oder Gemisch von Gasen, der oberhalb im Reaktionszylinder gebildeten partikelförmigen Siliciumpartikel erfolgt.

Eine weitere bevorzugte Ausführungsform des erfindungsgemäßen Verfahrens beinhaltet das Einleiten von Edelgas oder von Edelgasgemischen als Verdünnungsgas in das nicht-thermische Plasma, das insbesondere Temperaturen von kleiner gleich 1500 °C, vorzugsweise kleiner gleich 1300 °C, bevorzugt kleiner gleich 1100 °C aufweist.

Ein besonderer Vorteil des erfindungsgemäßen Verfahrens ist die effiziente Abtrennung der Partikel von gasförmigen Komponenten, wie dem Verdünnungsgas oder nicht umgesetzten reaktiven Gasen. So erfolgt vorzugsweise die Abtrennung der Partikel, indem diese durch eine gasdurchlässige Membran aufgefangen werden. So können die Partikel optional kontinuierlich oder diskontinuierlich über eine abschüssig angeordnete Membran abgetrennt werden. Das Verdünnungsgas kann mithilfe der Membran abgetrennt, aufgearbeitet und erneut verwendet werden.

In dem Verfahren nach der Erfindung werden vorzugsweise bei erhöhter Temperatur gasförmige Siliciumverbindungen umgesetzt, die umfassen Wasserstoff- und/oder Halogen-enthaltende Silane, wie H-Silane, Halogensilane, und/oder Kohlenwasserstoff-enthaltende Silane, wie Methylsilan, Methyltrichlorsilan, Dimethyldichlorsilan, Halogensilane, bevorzugt können auch Polyperchlorsilangemische umfassend Polyperchlorsilane mit 2 bis 8 Siliciumatomen eingesetzt werden. Besonders bevorzugt ist auch die Verwendung höhermolekularer Polychlorsilane mit mindestens drei Siliciumatomen, insbesondere mit 3 bis 8 Siliciumatomen. Zur Bestimmung der Anteile mit den vorgenannten Elementen und Verbindungen erfolgt vorzugsweise die Analyse mittels GC und/oder ICP-MS.

Vorzugsweise wird in dem Verfahren ein inertes Verdünnungsgas verwendet. Je nach Anforderungsprofil kann auf das Verdünnungsgas verzichtet werden, wenn zugleich ein Vakuum anliegt. Bevorzugte Verdünnungsgase sind Argon, Helium, Xenon, Krypton oder ein Gemisch umfassend mindestens zwei der genannten Gase. Stickstoff ist unter den genannten Reaktionsbedingungen kein inertes Verdünnungsgas, sondern ein reaktives Gas.

Die Zugabe von Edelgasen, insbesondere Argon und Xenon im Verfahren begünstigen die Bildung sphärischer Partikel. In dem erfindungsgemäßen Verfahren werden die gasförmige Siliciumverbindung, das Verdünnungsgas und optional das reaktive Gas als Gesamtzusammensetzung von 100 Vol.-% in dem Verfahren eingesetzt. Dabei ist es besonders bevorzugt, wenn die Verdünnungsgase mindestens zu 0,0001 bis 99 Vol.-%, insbesondere von 0,0001 bis 60 Vol.-%, bevorzugt von 0,1 bis 1,0 Vol.-% Edelgase enthält, insbesondere Xenon und/oder Argon. Somit ist es bevorzugt von 0,0001 bis 99 Vol.-%, insbesondere von 0,0001 bis 60 Vol.-%, bevorzugt von 0,1 bis 1,0 Vol.-% Edelgase in dem Verfahren einzusetzen, insbesondere Xenon und/oder Argon.

Das Verdünnungsgas kann vorzugsweise mit der gasförmigen Silicium-Verbindung und/oder dem reaktiven Gas vor dem Einbringen in den Reaktionszylinder gemischt werden. Alternativ wird das inerte Verdünnungsgas zur Abkühlung der Zersetzungsprodukte, wie beispielsweise zur Abscheidung der Pulver zugegeben. Die Zersetzung der Silicium-Verbindung und optional des reaktiven Gases kann auch durch Überführen der in einen Hochvakuum-Bereich erfolgen. Als ein Hoch-Vakuum wird ein Vakuum kleiner gleich 0,01 bar, insbesondere kleiner gleich 0,001 bar, kleiner gleich 0,0001 bar erachtet.

Der Druckbereich des Verfahrens liegt üblicherweise bei 1 mbar bis 50 bar, insbesondere bei 1 mbar bis 10 bar, bevorzugt bei 1 mbar bis 5 bar. Je nach dem gewünschten Zersetzungsprodukt und/oder Legierung und, um die Bildung von Kohlenstoff enthaltenden Prozessgasen zu minimieren, kann das Verfahren auch in einem Druckbereich von 1 bis 50 bar erfolgen, bevorzugt bei 2 bis 50 bar, besonders bevorzugt bei 5 bis 50 bar. Dabei weiß der Fachmann, dass der zu wählende Druck ein Kompromiss zwischen Gasabführung, Agglomerierung und Reduktion der Kohlenstoff enthaltenden Prozessgase ist.

Bevorzugt einsetzbare reaktive Gase als Legierungs- und optional Dotierungsbestandteile umfassen Stickstoff enthaltende Verbindungen, Germanium enthaltende Verbindungen, Stickstoff (N₂), NH₃, Hydrazin, Stickstoffwasserstoffsäure, eine Bor enthaltende Verbindung, wie Diboran, Kohlenwasserstoffe, wie Methan, Butan, Propan, höchstreines Erdgas, aromatische Verbindungen, wie Toluol, insbesondere jeweils sauerstofffreie Verbindungen, mit der Maßgabe, dass sich bei der Zersetzung kein Wasser bildet. Bevorzugte reaktive Gase umfassen Kohlenwasserstoffe, wie Methan, Ethan, Propan, Butan, Gemische dieser etc., HCl, Kohlenwasserstoffe mit Stickstoff. Zur Bildung der Bor enthaltenden Zone einer Siliciumlegierung wird bevorzugt Diboran, Bortrichlorid oder einer andere leicht zersetzbare Borverbindung eingesetzt. Wasserstoff kann je nach Reaktionsbedingungen als Verdünnungsgas oder auch als reaktives Gas eingesetzt werden.

Das reaktive Gas wird in dem Verfahren zugeführt, insbesondere zur Ausbildung der Siliciumlegierung und/oder einer Zone einer Siliciumlegierung umfassend Elekntronendonatoren und/oder Elektronenakzeptoren der Partikel.

Das reaktive Gas kann auch in einer Plasmareaktionszone mit einer Dotierverbindung wie Diboran oder Borhalogenid, insbesondere Diboran in einem Plasma umgesetzt werden. Je nach Verfahrensführung werden die Dotierverbindungen bevorzugt zur Ausbildung einer zumindest teilweisen Zone der Submicropartikel und/oder der Cluster eingesetzt werden. Bevorzugt ist eine vollständige Beschichtung als Zone der Partikel und optional der Macropartikel.

Als reaktive Gase gelten auch mit Bor, Aluminium und/oder Phosphor, Antimon, Selen, angereicherte Gase oder Gasgemische, die in Aufreinigungsprozessen zur Herstellung höchstreiner Verbindungen als Nebenstrom erhalten werden. Diese reaktiven Gase können bei konstanten Gehalten an Bor, Aluminium, Antimon, Phosphor etc., die hier als Dotierkomponente verwendet werden, zur Herstellung der dotierten und/oder beschichteten Siliciumpulver eingesetzt werden.

Die Bildung der Siliciumlegierung kann im Gleichstromplasma durch chemische Gasphasenabscheidung erfolgen. Generell kann die thermische Behandlung die Zersetzung umfassen sowie eine thermische Nachbehandlung, die zunächst ein starkes Abkühlen auf vorzugweise kleiner gleich 1200 °C zur Ausbildung der kristalliner Partikel umfasst, die anschließend in einem weiteren Verfahrensschritt zumindest teilweise in einem Plasma oder in einer thermischen Behandlung beschichtet werden können. Zusätzlich oder alternativ kann durch eine thermische Nachbehandlung auch eine Ausbildung der Cluster erfolgen und deren Geometrie gesteuert werden.

Die Siliciumverbindungen können mittels mindestens einer Düse in die Gasphase überführt werden oder als gasförmige Siliciumverbindung optional als Gemisch umfassend das reaktive Gas und/oder das inerte Verdünnungsgas in den Reaktionsraum eingebracht werden.

Zur Durchführung des Verfahrens ist es zusätzlich zu den vorgenannten Merkmalen weiter bevorzugt, wenn die Gasentladung ein nicht-thermisches Plasma ist, weiter bevorzugt erfolgt die Gasentladung in einer dielektrischen Barriereanordnung. Für die Definition des nichtthermischen Plasmas und der Partikelgenes wird auf die einschlägige Fachliteratur verwiesen, wie beispielsweise auf "Plasmatechnik: Grundlagen und Anwendungen - Eine Einführung; Autorenkollektiv, Carl Hanser Verlag, München/Wien; 1984, ISBN 3 446-13627-4".

Besonders bevorzugt beträgt die spezifische Energieflußdichte von 0,1 und 10 Ws/cm². Dabei ist es weiter bevorzugt, wenn der Energieeintrag mittels phasengenauer Momentanleistungsmessung mit einer Bandbreite von mindestens 250 kHz durchgeführt wird, wobei die Bestimmung der Momentanleistungsmessung in einem koaxialen Standard-Reaktor mit 50 cm² Entladungsfläche erfolgt. Ein koaxialer Reaktor ist vorzugsweise ein Rohrreaktor, insbesondere rotationssymmetrischer Rohrreaktor.

Der Energieeintrag zur Ausbildung des nicht-thermischen Plasmas erfolgt vorzugsweise in der Art, dass sich in dem sich ausbildenden Plasma möglichst homogene Bedingungen für die Umsetzung der Silane selbst bspw. mit und/oder ohne Ge, C, N, etc. enthaltenen Verbindungen ausbilden, dabei ist es besonders bevorzugt, wenn das nicht-thermische Plasma bei einer Spannung betrieben wird, bei der die Entladung die gesamte Elektrodenfläche bedeckt (normale Glimmentladung).

Gegenstand der Erfindung ist auch ein Reaktor (Freiraum Reaktor) **1** zur Herstellung von Siliciumpulvern mit Submicropartikeln deren Partikelgröße bei 5 nm bis 100 nm liegt, wobei der Reaktor **1** einen Reaktionszylinder **2** mit Reaktionsraum **2.1** umfasst und im Bereich des Reaktoranfangs **2a, 3** mindestens zwei Zuführungen **4.1, 4.2, 4.3** aufweist für die Einleitung
a) der mindestens einen bei erhöhter Temperatur gasförmigen Siliciumverbindung, und
b) optional des mindestens eines reaktiven Gases und/oder Verdünnungsgases, in den Reaktionsraum **2.1,** wobei dem Reaktionsraum **2.1** zumindest in einem Bereich am äußeren Umfang des Reaktionsraumes **2.1** Heizvorrichtungen 7 zugeordnet sind, und/oder dem Reaktionsraum **2.1** mindestens ein Plasmagenerator **9** zugeordnet ist, zur Erzeugung eines Plasmas im Reaktionsraum **2.1,** und wobei dem Reaktor 1 mindestens eine, vorzugsweise zwei Zuführungen **4, 4.1, 4.2** und/oder **4.3** im Bereich des Reaktoranfangs **2a** oder **3** zugeordnet sind. Nach der Alternative mit zwei Zuführungen sind die Zuführungen bevorzugt in einem Winkel von größer gleich 30° zueinander angeordnet. Im Reaktionsraum **2.1** ist mindestens eine Reaktionszone **2.1a,** alternativ sind mehrere Reaktionszonen **2.1a** bis **2.1n** im Reaktionsraum angeordnet. Vorteil der verschiedenen Reaktionszonen ist die Möglichkeit eine definierte Temperaturführung über die Reaktorläge einzustellen.

Vorzugsweise weist die Zuführung **4** einen Winkel von 0 bis 45° zur Mittelachse des Reaktionszylinders **2** auf, vorzugsweise 0 bis 10° und die optionale zweite Zuführung **4.3** einen Winkel von größer 45 bis 135° zur Mittelachse des Reaktionszylinders **2,** insbesondere um 80 bis 100°, bevorzugt um 90°. Die zweite Zuführung kann auch zur Einstellung der Strömung der gasförmigen Siliciumverbindung mit einem Verdünnungsgas optional im Gemisch mit einer Siliciumverbindung zudosiert werden.

Optional kann der Reaktor von dem Reaktoreingang und Reaktorausgang über weitere Zuführungen verfügen, die für die Bildung einer Legierung und/oder Ausbildung von Zonen einer Beschichtung der Siliciumpulver verwendet werden können. So kann beispielsweise über eine Zuführung im mittleren Bereich des Reaktorzylinders eine Beschichtung unter Ausbildung einer Zone auf den Partikeln erfolgen, indem beispielswiese eine Bor enthaltenden Verbindung in den Reaktionszylinder, insbesondere in eine Reaktionszone des Reaktionsraumes zugeführt wird.

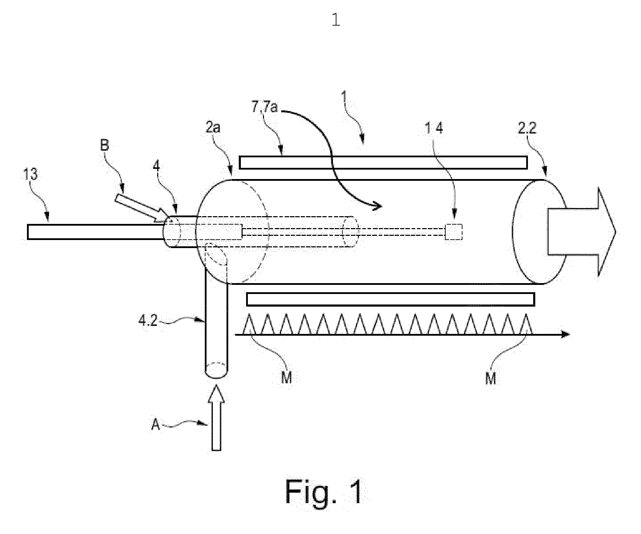

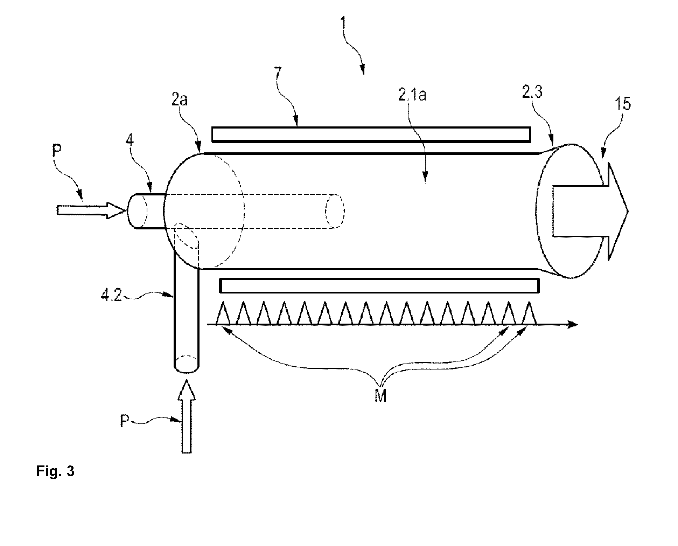

Der Reaktionsraum und/oder die mindestens eine Reaktionszone können zudem gemäß **Figur 1** und **3** einen Reaktoreinsatz mit Milli-Reaktionskanälen aufweisen. Der Reaktoreinsatz mit Millireaktionskanälen kann aus bevorzugt hochtemperaturstabilen Material, bspw. Siliciumcarbid gefertigt sein Anderen geeignete Materialien sind bevorzugt neben SiC, bspw. Siliciumnitrid, Korund (Al₂O₃), AIN, und/oder Graphit. Der Reaktoreinsatz dient dazu eine sehr schnelle Zersetzung und sehr schnelle Abscheidung über die Einstellung von Temperaturdifferenzen über sehr kurze Wegstrecken im Reaktionsraum und damit in den einzelnen Reaktionszonen zu gewährleisten.

Nach einer alternativen bevorzugten Ausführungsform weist der Reaktionsraum **2,** insbesondere in mindestens einer Reaktionszone **2.1a,** eine Reaktoreinsatz **16** mit Kanälen auf, wobei der Reaktoreinsatz vorzugsweise innere Kanälen umfasst, deren Durchmesser 1 bis 20 mm betragen, vorzugsweise 1 bis 10 mm, vorteilhaft sind die Kanäle rohrförmig, wobei ein rohrförmiger Kanal im Querschnitt jede beliebige Geometrie umfassend kreisförmig, wabenförmig etc. aufweisen kann.

Vorteilhaft verfügt der Reaktionsraum **2.1** über mindestens eine Reaktionszone **2.1.a** bis **2.1.n,** insbesondere über 2 bis 100 Reaktionszonen. In jeder Reaktionszone können definierte thermische Bedingungen oder Plasmabedingungen eingestellt werden.

Der Reaktor verfügt vorzugsweise im Bereich des Reaktorausgangs **2.2, 2.3** über eine Vorrichtung für die Auftrennung von Reaktionsprodukten, insbesondere für die Abtrennung des festen partikelförmigen Siliciumpulvers, vorzugsweise eine Vorrichtung zur Partikelabscheidung 15, bevorzugt eine Vorrichtung umfassend eine gasdurchlässige Membran **10** (nicht in den Figuren dargestellt). Der Reaktionsraum ist evakuierbar, so dass ein definiertes Vakuum einstellbar ist.

Das Plasma in der Entladungszone kann durch Hochspannungspulse in einfacher Weise als Plasma im nichtthermischen Gleichgewicht erzeugt werden. Die Temperaturen der Schwerteilchen (Atome, Ionen) sowie der Leichtteilchen (Elektronen) richtet sich nach reaktionstechnischen Erfordernissen.

Für die Abscheidung der Siliciumpulver wurde in einer alternativen Ausführungsform mindestens ein Funktionsmolekül optional ein inertes Verdünnungsgas zur Quenchen im Bereich des Reaktorausgangs des Reaktionszylinders eingesprüht. Über die Temperaturdifferenz und das zugeführte Gasvolumen an Funktionsmolekül kann die Partikelgrösse und die Abscheidungsrate eingestellt werden. Durch Zugabe reaktiver Gase oder organofunktioneller Silane kann hier auch eine Beschichtung, Legierungsbildung und/oder Funktionalisierung der Siliciumpulver erfolgen.

Gegenstand der Erfindung ist auch ein Verfahren in dem die (i) thermische Zersetzung zumindest teilweise an einer heißen Innenzone in einem Reaktionsraum **2.1** und/oder (ii) im Plasma in einem Reaktionsraum **2.1** in einem Reaktor **1** (Freiraum Reaktor), umfassend einen Reaktionszylinder **2** erfolgt, in dem im Bereich des Reaktoranfangs **2a, 3** in den Reaktionsraum **2.1** eines Reaktionszylinders 2 über mindestens eine Zuführung **4.1, 4.2, 4.3** die Siliciumverbindung und optional das Verdünnungsgas in den Reaktionsraum 2.1 eingebracht wird, der Reaktionsraum **2.1** umfasst mindestens eine Reaktionszone **2.1a bis 2.1n,** in mindestens einer Reaktionszone erfolgt die Zersetzung der Siliciumverbindung und optional die Bildung der Siliciumpulver, und am Reaktorausgang **2.2, 2.3** werden die gebildeten Submicropartikel und/oder Macropartikel in Form von Clustern der Submicropartikel erhalten.

Ebenfalls Gegenstand der Erfindung sind Formulierungen enthaltend Siliciumpulver erhältlich nach dem erfindungsgemäßen und mindestens einen Hilfsstoff und/oder ein Bindemittel und optional ein Lösemittel. Bevorzugte Formulierungen können vorliegen in Form einer Suspension, Dispersion oder einer Einbettung, einer Matrix, einer Formulierung, pharmazeutischen Formulierung, einer Bremspaste, Schleifpaste, Druckpaste, Tinte, in druckbaren Polymeren, elektrisch leitenden Paste, Haftklebemasse, eingebettet in eine Folie oder beispielsweise auch als Gründling vorliegen.

Das Siliciumpulver kann auch in gepresster, extrudierter, granulierter, gesinterter und/oder kristallisierter Form vorliegen und entsprechend abgepackt werden.

Die Formulierung kann in Form von Presslingen, als Formulierung vorliegen. Eine Formulierung kann eine Creme, ein Fluid sein, dass optional Siliciumpulver oder oberflächlich mit SiO₂ beschichtete Siliciumpulver als Sonnenschutz-Komponente umfasst. Mit SiO₂ beschichtete Siliciumpulver bilden sich durch Oxidation der Siliciumpartikel.

Gegenstand der Erfindung ist auch eine Formulierung enthaltend ein Siliciumpulver, insbesondere Silicium Partikel mit einem Hilfsmittel, Lösungsmittel und/oder Dispersionsmittel und/oder Suspensionsmittel.

Die erfindungsgemäßen Siliciumpulver eignen sich aufgrund ihrer Inertheit und ihres Transmissionsverhaltens besonders gut als UV-Schutzmittel, da die erfindungsgemäßen Pulver transparent vorliegen können und vorteilhaft ab 400 nm zu kurzen Wellenlängen hin keine nennenswerte Transmission aufweisen.

Gegenstand der Erfindung ist auch die Verwendung der Siliciumpulver sowie die nach dem Verfahren erhältlichen Siliciumpulver zur Herstellung von Thermoelementen, Halbleitern, Halbleitermaterialien, Solarsilicium, Halbleitersilicium, Pasten, Cremes, Coatingprodukten, Klebern, pharmazeutischen Produkten, Silicium enthaltenden Pasten (Solarindustrie, Beschichtung), Elektroden, Membranen, Katalysatoren, von LEDs, Displays, transparenter Schichten, Flat Panel Displays, Schmelztiegeln, Tiegeln zur Kristallisation hochreiner metallischer Verbindungen, Beschichtungen, Aerosolen, Materialien für die Thermoelektrik, zur Herstellung hochreiner polykristalliner Metallverbindungen oder von Einkristallen der Metallverbindungen, in Hochtemperatur Transistoren, die bei 100 bis 400 °C, vorzugsweise um 300 °C betrieben werden können, zur Herstellung von Hochtemperatursperrschichten, die bei 300 bis 400 °C betrieben werden können, Zur Herstellung von Hochtemperaturmembranen, die bis 400 °C betrieben werden können, zur Herstellung von Hochtemperaturbauteilen in Batterien, die bei 300 bis 400 °C betrieben werden können, zur Herstellung von Bauteilen, deren Absorption bei 300 bis 1000 nm sich im Wesentlichen linear ändert, insbesondere von Bauteilen, deren Transmission bei etwa 1000 nm 50% beträgt und bis 450 nm oder bis etwa 300 nm 20% beträgt und ab 300 nm im Wesentlichen keine Transmission mehr erlaubt, oder als Reaktand bei der Herstellung von kristallinem Silicium oder kristallinen Siliciumverbindungen, hochreine Schleifmittel, hochreine Hitzekacheln oder als Material von Artikeln. Ebenfalls Gegenstand der Erfindung ist die Verwendung der Siliciumpulver zur Herstellung von LEDs, Halbleitermaterialien, Flat Panel Displays und zur Herstellung von Materialien für die Thermoelektrik oder zur Herstellung von Einkristallen, Schleifmitteln, Isolatoren, als Feuerfeststoff, wie als Hitzekachel, oder bei der Herstellung von Artikeln oder bei der Herstellung von Elektroden und/oder Schmuck. Verwendung zur Herstellung von Halbleiterausgangsstoffen, Halbleitern, Thermoelementen zur Energierückgewinnung aus Abwärme, insbesondere von hochtemperaturstabilen Thermoelementen, thermoelektrischen Komponenten, wie thermoelektrischen Generatoren (TEG) sowie zur Herstellung von Hochtemperatursperrschichten und UV/Vis-transparenten und hochtemperaturstabilen Bauteilen, und/oder zur Herstellung von OLEDs verwendet werden, zur Herstellung von Beschichtungen von Tiegeln (Crucibles).

Gegenstand der Erfindung ist die Verwendung der Siliciumpulver, umfassend Submicropartikel mit einer Partikelgröße von 5 bis 3000 nm, insbesondere von 5 bis 30 nm, bevorzugt von 5 bis 20 nm, wobei die Submicropartikel im Wesentlichen kristallin sind, insbesondere weist das Siliciumpulver eine Kristallinität von größer gleich 85 % auf, zur Herstellung von Beschichtungen von Tiegeln/Crucibles. Mit den erfindungsgemäßen Siliciumpulvern ist eine Einsparung von 20% der Verfahrenszeiten bei der Herstellung von Beschichtungen von Tiegeln/Cruciblen möglich. Die Tiegel werden in der Solarindustrie zur Herstellung von Silizium mit Solargrade eingesetzt. Bislang werden nach einem Verfahren die Tiegel/Crucibles nach dem Panasonic Verfahren (US2013/0276694A1) innen mit reinem amorphem Silizium beschichtet. Durch Verwendung der kristallinen Siliciumpulver anstatt der amorphen Siliciumpulver können die Prozesszeiten im Beschichtungsprozess der Tiegel somit deutlich reduziert werden.

Die Partikelgrößenbestimmung der Siliciumpulver als auch die Bildung von Agglomeraten wie die Macropartikel oder das Vorliegen von Submicropartikel kann analytisch mit nachfolgenden Methoden bestimmt werden. Die Bestimmung der Teilchengröße kann u.a. mittels Siebanalyse, TEM (Transelektronenmikroskopie), REM (Rasterkraftelektronenmikroskopie) oder Lichtmikroskopie erfolgen.

Bevorzugt liegt die Leitfähigkeit (vgl. http://de.wikipedia.org/wiki/Elektrische Leitfähigkeit) der Verfahrensprodukte, insbesondere der hochdicht verpreßten und versintertem pulvrigen Verfahrensprodukte, gemessen in der dem Fachmann geläugigen Art zwischen zwei spitzen Elektroden von π [m/Ω.m²] = 1 • 10¹ bis 1 • 10⁻⁶. Angestrebt wird für das jeweilige Materialkörper bei 200 °C bspw. für Silizium-Bor-Legierung 1 • 10 S/m, SiGe 8 S/m, SiN < 1 • 10⁻⁶ S/m , SiC< 1 • 10⁻³. Die Leitfähigkeit korreliert direkt mit der Reinheit des Verfahrensproduktes.

Die nachfolgend erläuterten Figuren und Ausführungsbeispiele erläutern exemplarisch die Gegenstände der Erfindung ohne diese auf diese konkreten Beispiele zu beschränken.

### Ausführungsbeispiele

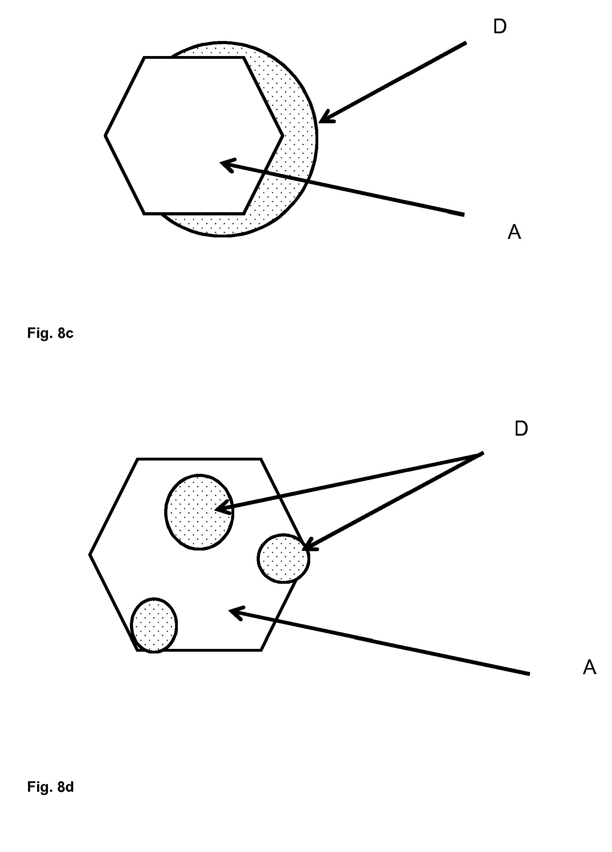

Die nachfolgenden Figuren stellen dar:
**Figur 1****:** Freiraum Reaktor mit Abschirmrohr **2.** Freiraum-Reaktor (FSR)
**Figur 2a****:** Polykristalline Submicropartikel von Silicium (200000 : 1; Auflösung 200 nm). Ein exemplarischer Partikel ist 77,45 nm groß.
**Figur 2b****:** Vergrößerung der **Figur 2a** (600000 : 1; Auflösung 50 nm).
**Figur 3****:** Hot-Wall-Reaktor bestehend aus einem Heraeus Ofen ROF 4/50 mit Ofenrohr und Mikrokanal-Lanze und schraubenförmiger Wandströmung

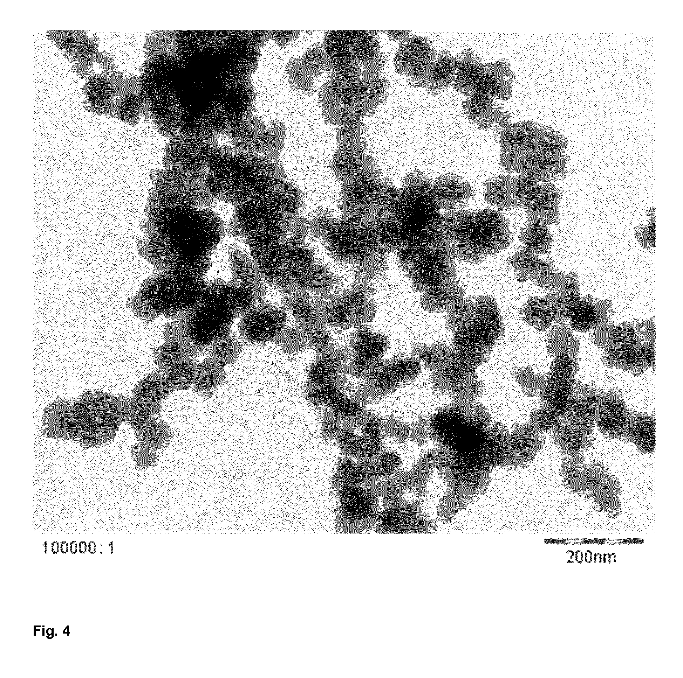

**Figur 4****:** TEM-Aufnahme einer typischen Probe von Siliziumnanopartikeln aus dem Heißwandreaktor (100000 : 1; Auflösung 200 nm).
**Figur 5****:** TEM-Aufnahme einer typischen Probe von Siliziumnanopartikeln aus dem Heißwandreaktor (100000 : 1; Auflösung 200 nm).
**Figur 6a****:** Reine kristalline Siliciumpartikel (Bereich A, EDX-Analyse);
**Figur 6b****:** HR-TEM-Aufnahme eines Siliziumnanopartikelausschnitt (eingezeichneter Abstand: 0,31 nm).
**Figur 7****:** Beugungsaufnahme einer Fourier-Transformation des Kernbereichs des Teilchens.

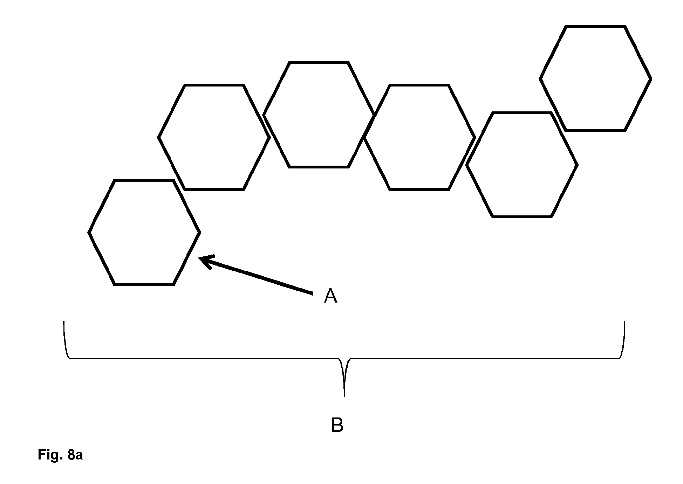

**Figur 8a****:** Schematisches Darstellung von Submicropartikeln **A** mit einer kristallinen Phase, die agglomeriert als Cluster in Macropartikeln vorliegen.

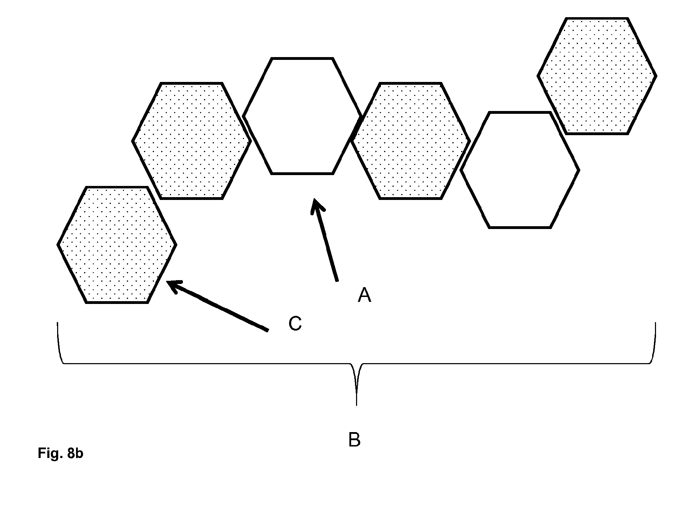

**Figur 8b** zeigt schematisch Submicropartikel **C** mit einer Siliciumlegierung, die kristallin oder ggf.
amorph vorliegen kann sowie Submicropartikel **A** mit einer kristallinen Siliciumphase. **Figuren 8c** und **8d** stellen schematisch eine kristalline Siliciumphase aufweisenden Submicropartikel **A** mit mindestens einer Zone **D einer** Siliciumlegierung eines Elektronenakzeptors oder
Elektronendonators dar, mit eine zumindest teilweisen äußeren Beschichtung oder mit in den Submicropartikeln gebildeten Zonen **D.** Bevorzugt sind Siliciumlegierungen in C und D mit Bor.
Erfindungsgemäße Siliciumpulver können die Submicropartikel der **Figuren 8c, 8d** als Macropartikel nach **Figur 8b** umfassen.

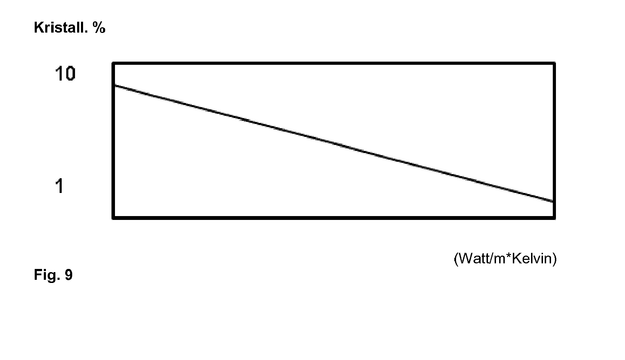

**Figur 9****:** Darstellung der Wärmeleitfähigkeit von kristallinen (Kristallinität 16 bis 99%) mit Phosphor dotierten Siliciumsubmicropartikeln einer Partikelgröße von etwa 5nm.

**Figur 1** zeigt einen Freiraum Reaktor **1** mit Abschirmrohr **2.** Freiraum-Reator (FSR) mit tangentialer Wandströmung. Die Anordnung zur Temperatur-Messung im Ofen und die Geometriedaten sind nachfolgend zusammengefasst **(2a:** Reaktor-Rohr: Aussendurchmesser: 36,1 mm; Innendurchmesser: 33,1 mm **7a:** Heizzone Länge 700 mm bei T = 1600 °C; **4:** Abschirmrohr für Lanze: Aussendurchmesser: 6,7 mm, Innendurchmesser: 3,7 mm; **13:** Temperatur-Messlanze mit Meßfühler; **14:** Temperaturmessfühler für Hochtemperatur-messung bis 2000°C); **2.2:** Reaktor-Rohr Ausgang; **4.2:** Zuführung tangential für Zirkulargasströmung im Reaktor; **A:** Prozessgas A; **B:** Prozessgas B.

**Figur 3** zeigt einen Hot-Wall-Reaktor bestehend aus einem Heraeus Ofen ROF 4/50 mit Ofenrohr und Mikrokanal-Lanze und schraubenförmiger Wandströmung von inerten Gasen. Dies ist ein Reaktor für die Herstellung von nanokristallinem Silizium mit Oberflächenausscheidungen und/oder Oberflächenabscheidung von Dotierelement z.B. Bor. Anwendungsfeld thermoelektrisch aktives Material. Ofen Geometriedaten: **2a:** Reaktor-Rohr: Aussendurchmesser: 36,1 mm; Innendurchmesser: 33,1 mm; **7:** Heizzone Länge 700 mm bei T = 1250 °C oder 1600 °C; **15:** Partikelabscheidung; **4:** Abschirm-Lanzenrohr (Aussendurchmesser: 6,7 mm, Innendurchmesser: 3,7 mm; verschiebbar: 31 cm in Ofenzone); **2.1a:** Reaktionszone 1250 °C bzw. 1600 °C; **2.3:** Reaktor-Rohr Ausgang (mit Venturi-Trichter) mit Quenchzone; **4.2:** Zuführung tangential für Zirkulargasströmung im Reaktor; **A:** Prozessgas A z.B. Monosilan; **B:** Prozessgas B z.B. Diboran.

Die Plasmaentladung kann über verschiedenartige Wechselspannungen oder gepulste Spannungen von 1 bis 10⁶ V angeregt werden. Weiterhin kann der Kurvenverlauf, der für die Erzeugung der Entladung angelegten Spannung beispielsweise - aber nicht ausschliesslich - rechteckig, trapezförmig, sinusförmig, dreieckförmig, gepulst oder stückweise aus einzelnen zeitlichen Verläufen zusammengesetzt sein. Weiterhin kann die Erzeugung geeigneter zeitlicher Kurvenverläufe durch Fourier-Synthese erfolgen.

Für eine hohe Elektronendichte und möglichst gleichzeitige Ausbildung der Entladung im gesamten Entladungsraum des Reaktors sind pulsförmige Anregungsspannungen besonders geeignet. Die Impulsdauer bei Pulsbetrieb richtet sich nach dem Gassystem und liegt bevorzugt bei 10 ns bis 1 ms. Die Spannungsamplituden können 10 Vp bis 100 kVp, bevorzugt 100 Vp bis 10 kVp, besonders bevorzugt 500 Vp bis 5 kVp, in einem Mikrosystem betragen. Diese gepulsten Gleichspannungen können auch von hohen Wiederholraten, beispielsweise von 10 MHz im Fall der 10-ns-Impulse (Tastverhältnis 10 : 1) bis zu niedrigen Frequenzen hin (10 bis 0,01 Hz), gefahren und moduliert werden, beispielsweise als "Burstfunktionen", um die Reaktion von adsorbierten Spezies zu ermöglichen.

### Bezugszeichenliste:

- **1**: Reaktor (Freiraum Reaktor)
- **2**: Reaktionszylinder (horizontal oder vertikal),
- **2a**: horizontaler Reaktionszylinder
- **2.1**: Reaktionsraum; **2.1a:** Reaktionszone, Reaktionszone **2.1a** bis **2.1n**
- **2.2**: Reaktorzylinder Ausgang, insbesondere Reaktorrohr Ausgang
- **2.3**: Reaktorzylinder Ausgang (mit Venturi-Trichter) Quenchzone
- **3**: oberhalb **3** im Reaktionsraum **(2.1)**
- **4**: Abschirmung für Lanze; **4.1:** mindestens einer Düse
- **4.2**: Zuführung tangential, insbesondere für Zirkulargasströmung im Reaktor
- **4.3**: Prozessgaszuführung mit Gasmischeinheit
- **5**: untere Bereich im Reaktor **2.1**
- **6**: Vorrichtung
- **7**: Heizvorrichtung; **7a:** Heizzone
- **8**: Reaktorwand
- **9**: Plasmagenerator
- **10**: gasdurchlässige Membran
- **11**: Düse(n)
- **12**: rotierende Vorrichtung
- **13**: Temperatur-Messlanze
- **14**: Temperatur-Messfühler
- **15**: Partikelabscheidung
- Ein **16**: Reaktoreinsatz mit Milli-Kanälen z.B. SiC-Fritte oder mit **16.1** Millikanal Ausschnitt sind nicht dargestellt,
- **P**: Prozessgas
- **M**: Messpunkte im Reaktor

**A:** Submicropartikel; **B:** Macropartikel, **C:** Siliciumlegierung; **D:** Zone einer Siliciumlegierung; **A:** Prozessgas A; **B:** Prozessgas B; **AB:** Wasserstoff/Ethan für Regelung Zusammensetzung Prozessgas / Wärmeübertrag/Partikelbildung im Reaktor; **AA:** Prozessgasmischung z.B. Monosilan-Ethan-Gemisch; **AAA** Vorgemischtes Prozessgas; **ABB** Wasserstoff/Argon für Regelung Zusammensetzung Prozessgas / Wärmeübertrag/Partikelbildung im Reaktor; **AAA:** Prozessgasmischung z.B. Monosilan-Wasserstoff-Gemisch.

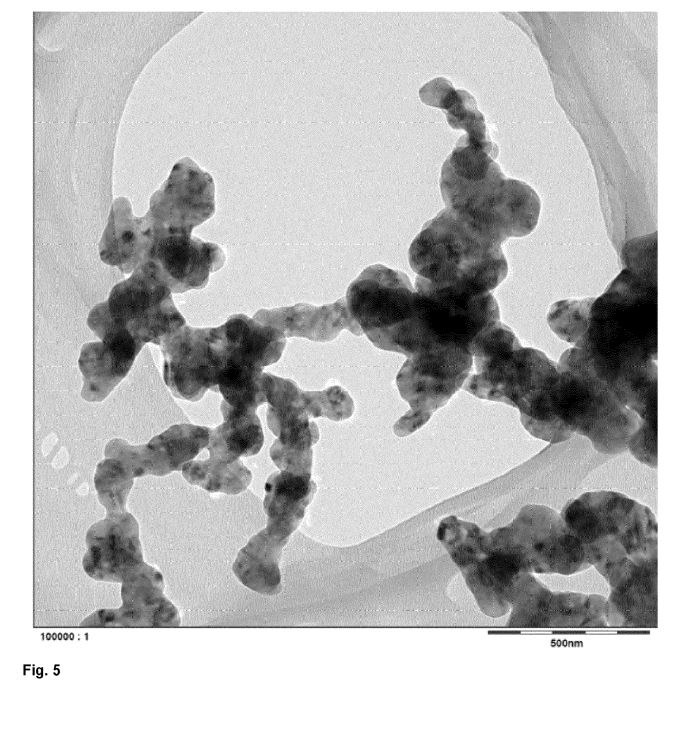

Alle Angaben in Liter entsprechen Normliter. Der Plasmareaktor wurde bei 0,45 kW und ca. 14 kHz mit Hochspannungspulsen mit einer Halbwertsbreite von t(50) 567ns und einem Massestrom von 43 NL/min betrieben. Für die nachstehend genannten Plasma-Verfahren wurden im nichtthermischen Gleichgewicht betriebene Gasentladungen verwendet.
Strukturelle Untersuchungen (REM, TEM, XRD) belegen die Nanostruktur der dargestellten Materialien eindeutig. Elektronenmikroskopische Untersuchungen erfolgen mit HR-TEM, TEM und SEM. Für die HR-TEM- und TEM-Untersuchungen wurde das Materialpulver in Aceton dispergiert und mit Ultraschall für 1 Stunde bei typisch 200 W Leistung beschallt. Die Dispersion wurde auf die TEM-Grids aufgetragen und für die elektronenmikroskopische Analyse vorbereitet. Figur 5 zeigt die TEM-Aufnahme einer typischen Probe von Siliziumnanopartikeln aus dem Heißwandreaktor. Die Partikel zeigen eine aggregierte Struktur bestehend aus kleineren Einheiten (Primärteilchen) mit einem Durchmesser kleiner als 100 nm, wobei ausgeprägte Sinterhälse zwischen benachbarten Primärteilchen erkennbar sind. Für die Auswertung wurden verwendet:

XRPD: PANalytical SAXS, Propenpräparation zwischen zwei Mylarfolien in einem Transmissionsprobenträger. Auswertungssoftware: SAXS140058. Die Partikel-größenbestimmung der Cluster erfolgt an Proben, die in Ethanol im Ultraschall für 5 Minuten dispergiert wurden (Ultrastab-Finger). Alternativ können die Cluster oder Submicropartikel-Größen durch Ausmessen der TEM-Proben bestimmt werden.

Es wurden Partikel in einem Druckbereich von 15 und 100 kPa in einer Gasentladung bei einer geschätzten Temperatur von 800 °C bis 1000 °C hergestellt. Die Partikelbildung erfolgte bei Prekursorkonzentrationen von bis zu von 10 Vol.% Silan in einer H₂/Ar-Atmosphäre bei einem anfänglichen Gesamtgasfluss von 10 slm, woraus sich nach der Zersetzung von SiH₄ ein Gasfluss von 11 slm ergibt. Die Prekursorkonzentration von 10 Vol.% SiH₄ ermöglicht eine Herstellungsrate von 0,07 kg/h. Bezüglich der Reaktorgeometrie und der Annahme einer konstanten Prozessgastemperatur von 1000 °C innerhalb des Reaktors, wird die Gasgeschwindigkeit mit 0,7 m/s bei 100 kPa und 4,8 m/s bei 15 kPa berechnet.

Für die BET-Analyse wurde das Siliziumpulver aus dem Filter entnommen und nach 24-stündigem Ausgasen im Vakuum bei 250 °C analysiert. Die BET-Ergebnisse zeigen, wie für die Gasphasensynthese erwartet, eine zunehmende spezifische Oberfläche mit abnehmendem Prozessdruck aufgrund des begrenzten Teilchenwachstums und der abnehmenden Verweilzeit während der Synthese. Die Teilchengröße nimmt mit steigenden Prozessdrücken von unter 100 nm auf 200 nm zu. Die folgende Tabelle zeigt die Ergebnisse im Überblick:

| Mittlere Partikelgröße | Druck | Flow/Volumenfluss | BET |
|---|---|---|---|
| 80 nm | 15 kPa | 11slm bei 4,8 m/s | 35m²/g |
| | | | |
| 210 nm | 100 kPa | 11slm bei 0,7 m/s | 15 m²/g |

| | | | |
|---|---|---|---|
| *slm: Standardliter/Minute | | | |

**Ausführungsbeispiel 1:** Gewinnung von oberflächenlegierten Siliciumpartikel hochreiner Form in einem Free-Space Reaktor. Es wird Monosilan zur Reaktion gebracht. Gemäß der allgemeinen Reaktionsgleichung

1 SiH₄ → Si + 2H₂

wird Silicium abgeschieden. In der Gasphase, an einer in Richtung Ausgang liegenden Einspeisestelle am FSR wird eine borhaltige Verbindung, z.B. Diboran eingedüst und im FSR thermisch zersetzt, wodurch Bor auf der Partikeloberfläche abgeschieden wird. Es werden 2 Normliter/Min Argon mit 10 ppm.-Vol Diboran und 10 ppm-Vol Xenon und 1 Normliter/Min Argon mit 5 Gew.-% SiH₄ eingetragen und bei einer Temperatur von 1600 °C mit einer Verweilzeit von 400 Millisekunden in einem Reaktor gemäß **Figur 1** oder **3** umgesetzt.

Das Produkt wird über einen am Ende des Reaktionsrohr angebrachten Konus einem Filter zugeführt, in welchem die Silicium-Borpartikel aufgehalten werden und isoliert werden. Kristallines Si mit unterschiedlichem Borgehalt auf der Oberfläche (z.B. 2 Gew-%) wurde bei ca. 1600 °C in H₂-Atmospäre hergestellt. Erhalten wurde dotiertes kristallines Siliciumpulver, das an der Oberfläche mit Bor angereichert ist. Das Pulver umfasst Submicropartikel mit einem typischen Durchmesser von 5 bis 10 nm.

**Ausführungsbeispiel 2:** Gewinnung von kristallinen Siliciumpartikeln in hochreiner Form in einem Free-Space Reaktor. In der Gasphase wird Monosilan im FSR thermisch zersetzt. Es werden 2 Normliter/Min Argon und 1 Normliter/Min Argon mit 5 Gew.-% SiH₄ bei einer Temperatur von 1600 °C mit Verweilzeit von 400 Millisekunden in einem Reaktor gemäß
**Figur 1** oder **3** umgesetzt. Das Produkt wird über einen am Ende des Reaktionsrohr angebrachten Konus einem Filter zugeführt, in welchem die Silicium-Partikel aufgehalten werden und isoliert werden können. Erhalten wurden Siliciumpulver umfassend Submicropartikel mit einem Partikeldurchmesser von 4 bis 11 nm.

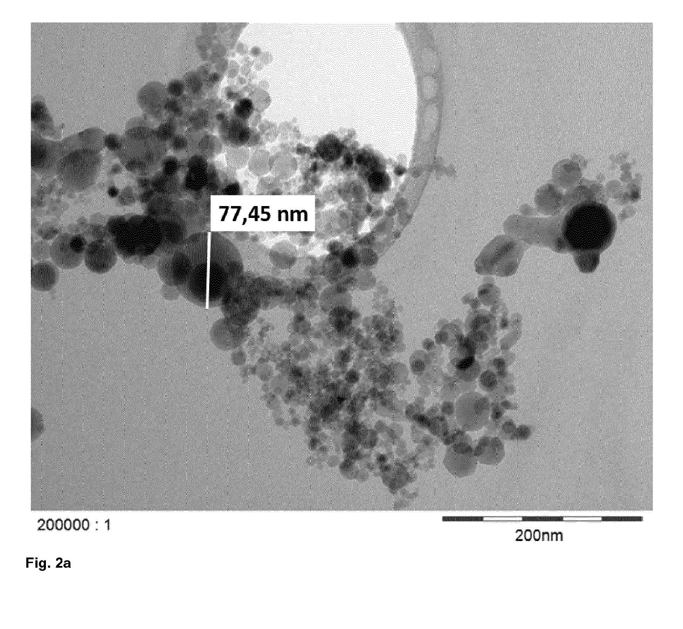

**Ausführungsbeispiel 3a:** Es werden 1 Normliter/Min Argon und 2 Normliter/Min Argon mit 5 Gew.-% SiH₄ bei Plasmaanregung bei einem Leistungsfluß von ca. 250 Watt und mit einer Verweilzeit von 10 Millisekunden umgesetzt. Es werden ebenfalls Siliciumpulver umfassend Submicropartikel und Makropartikel erhalten, deren Partikeldurchmesser im Durchschnitt bei etwa 40 bis 60 nm liegt. Die **Figur 2a** zeigt eine TEM-Aufnahme des kristallinen Si Pulvers (Präparation Ethanol verdünnt aufgetragen), die Submicropartikel von Silicium mit Submicropartikeldurchmessern von beispielsweise 77 nm und 45 nm zeigt.

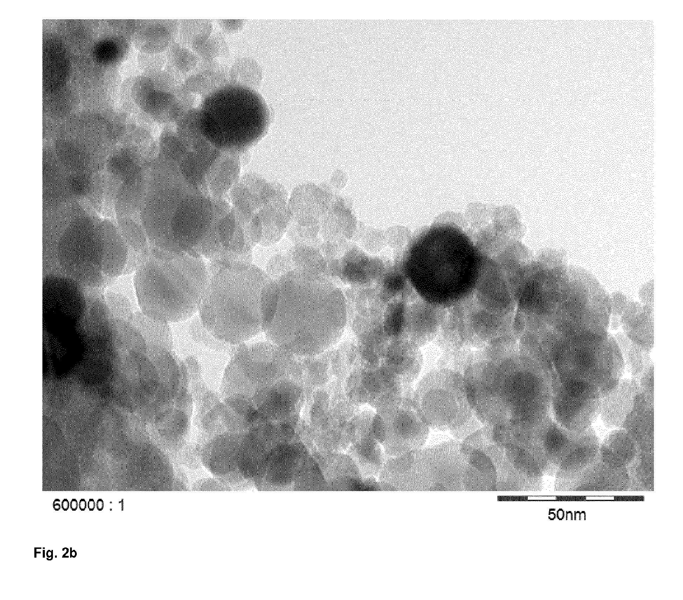

**Ausführungsbeispiel 3b:** Die Verfahrensführung war analog dem Beispiel 3a mit der Abweichung, dass die Verweilzeit 3 Millisekunden betrug. Die Figuren 2a und 2b zeigen polykristalline Submicropartikel von Silicium.

**Ausführungsbeispiel 4:** Gewinnung von nanokristallinen Siliciumpartikeln deren Submicropartikel eine Partikelgröße von etwa 150 nm aufweisen.

Gewinnung von Siliciumpartikeln mit großen Primärpartikeln im Bereich von 150 nm in hochreiner Form in einem FSR. Es wird Monosilan zur Reaktion gebracht. Gemäß der Reaktionsgleichung

1 SiH₄ → Si + 2 H₂

wird das Zielprodukt Silicium erhalten. In der Gasphase wird Monosilan im FSR thermisch zersetzt. Als Prozessgasfluss werden eingesetzt: 2 Normliter/Min Argon und 1 Normliter/Min Argon mit 5 Gew.-% SiH₄ bei 1600 °C, die bei einer Verweilzeit von rund 100 Millisekunden, (Reaktor **Figur 3****,** Hotwall) umgesetzt werden. Das Produkt wird über einen am Ende des Reaktionsrohr angebrachten Konus einem Filter zugeführt, in welchem die Silicium-Partikel aufgehalten werden und isoliert werden können. Polykristallines Si wurde bei ca. 1600 °C in H₂-Atmospäre hergestellt.

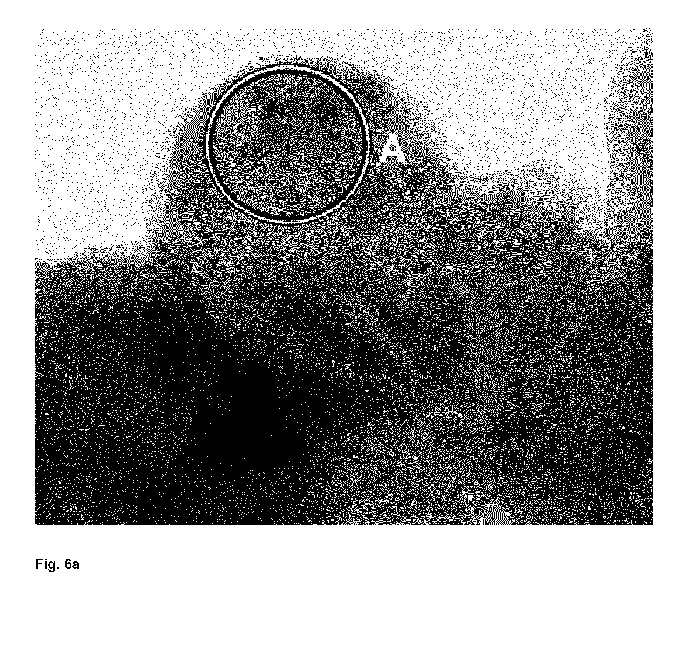

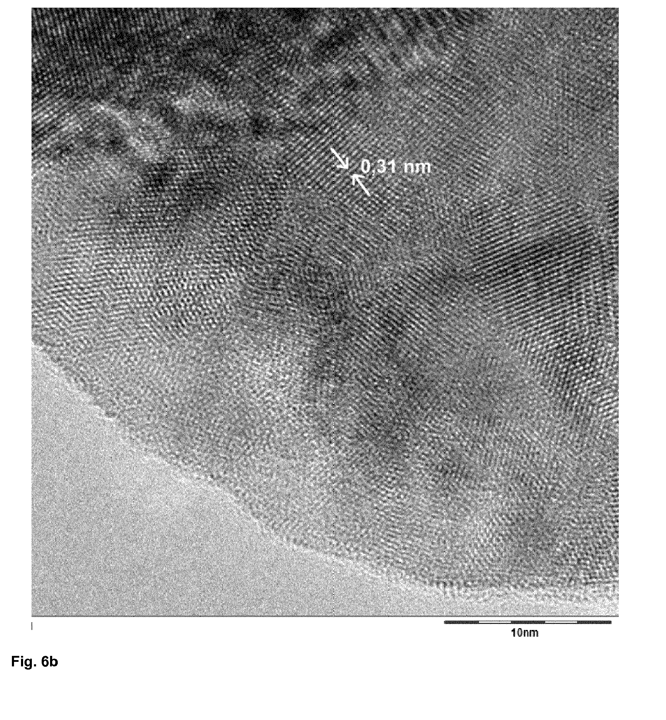

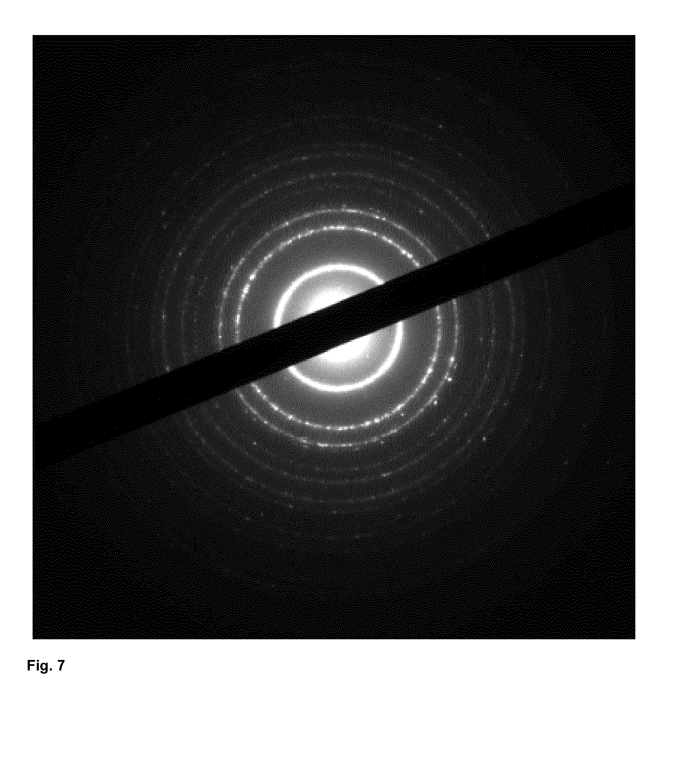

Die Probenpräparation der Si-Partikel für TEM-Analysen erfolgte mittels Dispergieren in Isopropanol/Wasser mit nachfolgendem Aufgetrocknen. **Figur 5** zeigt eine ATEM-Aufnahmen von kristallinem Silicium mit Macropartikel in Form von verzweigten Silicium dots und Ketten; Es zeigen ferner die **Figuren 6a** und **6b** nach dem Verfahren gewonnene reine kristalline Siliciumpartikel. **Figur 6a** zeigt reine kristalline Siliciumpartikel (Bereich A, EDX-Analyse) und die **Figur 6b** zeigt eine HR-TEM-Aufnahmen eines Siliziumnanopartikelausschnitt, mit reinen, einkristallienen, nanokristallinen Silicumpartikeln und auch einige Defekte und Stapelfehler im Kern des Teilchens, Gitterstruktur des Materials sowie Grenze des Teilchens mit dem TEM-Grid, ein ausgeprägte Oxidschicht ist nicht sichtbar. Die **Figur 7** zeigt ein Beugungsaufnahme einer Fourier-Transformation des Kernbereichs des Teilchens.

**Ausführungsbeispiel 5:** Gewinnung von oberflächenlegierten Siliciumpartikel in hochreiner Form in einem Freiraum-Reaktor. Es wird Monosilan zur Reaktion gebracht. Gemäß der Reaktionsgleichung

1 SiH₄ → Si + 2 H₂

wird das Zielprodukt Silicium erhalten. Es werden als Prozessgasfluss eingesetzt 2 Normliter/Min Argon und 1 Normliter/Min Argon mit 5 Gew.-% SiH₄ bei einer Temperatur von 1500 °C bei einer Verweilzeit 500 Millisekunden in den Reaktor der **Figur 2** eingetragen.

In der Gasphase, an einer in Richtung Ausgang liegenden Einspeisestelle am FSR wird eine borhaltige Verbindung, z.B. Diboran eingedüst, im FSR thermisch zersetzt wodurch Bor auf der Partikeloberfläche abgeschieden wird. Das Produkt wird über einen am Ende des Reaktionsrohr angebrachten Konus einem Filter zugeführt, in welchem die Silicium-Borpartikel aufgehalten werden und isoliert werden können. Nanokiristallines Si mit unterschiedlichem Borgehalt auf der Oberfläche (z.B. 2,5 Gew-%) wurde bei ca. 1500 °C in H₂-Atmosphäre hergestellt. Die Partikelgrößen der nanokristallinen Submicropartikel liegen bei 3 bis 14 nm.

**Ausführungsbeispiel 6:** Gewinnung von Silicium in hochreiner Form in einem Plasma-Reaktor. Es wird Monosilan zur Reaktion gebracht. Dazu werden 2 Normliter/Min Argon und 1 Normliter/Min Argon mit 5 Gew.-% SiH₄ eingetragen. Die Elektroden des Plasma Reaktors haben eine Schlagweite von 4 mm (Gap-Abstand) und werden bei einer Betriebsfrequenz des Vorschaltgerätes ca. 3,8 kHz mit bipolarem Impuls-Betrieb (Impulse von ca. 500 ns Implusdauer), Amplitude ca. 70 % ( etwa 10 kVp), Betriebsdruck 1040 bar abs. betrieben. Das Produkt wird über einen an das Reaktionsrohr angebrachten Konus einem Filter zugeführt, in welchem die Siliciumpartikel aufgehalten und isoliert werden können. Mit einer Verweilzeit von 1 bis 100 Millisekunden konnten monokristalline Silicium Submicropartikel hergestellt werden. Die Partikelgrößen der Submicropartikel liegen bei < 5 bis 10 nm.

**Ausführungsbeispiel 7:** Gewinnung von Silicium in hochreiner Form in einem Plasma Reaktor. Es wird Monosilan zur Reaktion gebracht. Es werden 2 Normliter/Min Argon und 1 Normliter/Min Argon mit 5 Gew.-% SiH₄ eingebracht. Die Elektroden des Plasma Reaktors haben eine Schlagweite von 4 mm (Gap-Abstand), eine Betriebsfrequenz des Vorschaltgerätes von ca. 3,8 kHz und es wird im bipolaren Impuls-Betrieb (Impulse von ca. 500 ns Implusdauer), Amplitude ca. 70 % ( etwa 10 kVp, Kilovolt Peak) bei einem Betriebsdruck 1040 bar abs. umgesetzt. Mit einer Verweilzeit von mehr als 30 Millisekunden konnten monokristalline Silicium Submicropartikel hergestellt werden. Die Partikelgrößen der Submicropartikel liegen bei 40 bis 60 nm.

**Ausführungsbeispiel 8:** Gewinnung von mit Phosphor dotierten Siliciumpartikeln in hochreiner Form durch Zugabe von Ammoniak in Gegenwart einer Phosphor enthaltenden Verbindung, wie PH₃, in einem Freiraum-Reaktor. Es wird Monosilan zur Reaktion gebracht. Gemäß der Reaktionsgleichung

1 SiH₄ → Si + 2 H₂

werden kristalline Submicropartikel umfasst und die Submicropartikel eine Partikelgröße von etwa 5 nm Silicium erhalten. Es werden als Prozessgasfluss eingesetzt
2 Normliter/Min Argon und 1 Normliter/Min Argon mit 5 Gew.-% SiH₄ bei einer Temperatur von 1500 °C bei einer Verweilzeit 500 Millisekunden in den Reaktor der **Figur 2** in Gegenwart von NH₃ und PH₃ eingetragen.

Nanokristallines Si mit einer guten Verteilung des Dotier-Phosphors wurde bei ca. 1500 °C in H₂-Atmosphäre hergestellt. Die Partikelgrößen der nanokristallinen Submicropartikel liegen bei etwa 5 nm. Durch Variation der Verweilzeiten und/oder der Reaktortemperatur konnte der Anteil an Kristallinität der Siliciumsubmicropartikel von 15% bis nahezu 100 % (99,9%) variiert werden. **Figur 9** stellt dar die Abhängigkeit der Wärmeleitfähigkeit (Y-Achse: rel. Einheiten (Watt/m*Kelvin)) von der Kristallinität (X-Achse: Kristallinität 16% bis 99%) von mit Phosphor dotierten Siliciumsubmicropartikeln einer Partikelgröße von etwa 5nm.

Es wurde phosphordotiertes nanokristallines Silizium erhalten, dass durch Zugabe von geringen Mengen an Ammoniak überwiegend als kristallines Si mit rund d₅₀ = 5 nm erhalten wurde. Die Zugabe des Ammoniaks bewirkt eine verbesserte Verteilung des Dotier-Phosphors und damit eine verbesserte halbleitende elektrische Leitfähigkeit. Dies führt zu einer verbesserten thermoelektrischen Performance.

## Patentansprüche

1. Siliciumpulver, **dadurch gekennzeichnet, dass** das Siliciumpulver Submicropartikel umfasst und die Submicropartikel eine Partikelgröße von 5 bis 3000 nm aufweisen, wobei die Submicropartikel im Wesentlichen kristallin sind, wobei optional das Siliciumpulver eine Kristallinität von größer gleich 15,5% bis 99,9 % aufweist.

2. Siliciumpulver nach Anspruch 1, **dadurch gekennzeichnet, dass** mindestens ein Submicropartikel eine Siliciumlegierung mit größer gleich 10²¹ Atomen/cm³ bis 6 · 10²² Atomen/cm³ ausgewählt aus mindestens einem Elektronendonator und Elektronenakzeptor aufweist.

3. Siliciumpulver nach Anspruch 1 oder 2, **dadurch gekennzeichnet, dass** die Submicropartikel in Clustern als Macropartikel vorliegen, wobei die Macropartikel eine Partikelgröße von 10 nm bis 3 µm aufweisen.

4. Siliciumpulver nach einem der Ansprüche 1 bis 3, **dadurch gekennzeichnet, dass** die Siliciumpulver umfassend Submicropartikel und optional Macropartikel eine Siliciumlegierung mit einem Element der Gruppe 13 oder 15 des Periodensystems der Elemente nach IUPC aufweisen und/oder zumindest eine Zone einer Siliciumlegierung mit einem Element der Gruppe 13 oder 15 aufweisen.

5. Siliciumpulver nach einem der Ansprüche 1 bis 4, **dadurch gekennzeichnet, dass**
a) die Submicropartikel aus einer Siliciumborlegierung bestehen, und/oder
b) die Submicropartikel mindestens eine Zone einer Siliciumborlegierung umfassen, insbesondere liegt die Zone als eine zumindest teilweise Beschichtung der Submicropartikel vor.

6. Siliciumpulver nach einem der Ansprüche 1 bis 5, **dadurch gekennzeichnet, dass** das Siliciumpulver thermoelektrische Eigenschaften aufweist.

7. Siliciumpulver nach einem der Ansprüche 1 bis 6, **dadurch gekennzeichnet, dass** die Submicropartikel ausgewählt sind aus Submicropartikeln, die
a) im Wesentlichen eine monokristalline Phase einer Siliciumlegierung umfassen, insbesondere eine Silicium und Bor enthaltende Legierung sowie optional mindestens eine Zone einer Silicium und Bor enthaltenden Legierung, und/oder
b) dotierte Domänen eine Siliciumlegierung mit kleiner 10²¹ Atomen/cm³ bis 10¹⁸ Atomen/cm³ umfassen, ausgewählt aus mindestens einem Elektronendonator und Elektronenakzeptor, wobei die Submicropartikel optional mindestens eine Zone einer Siliciumlegierung umfassen.

8. Verfahren zur Herstellung von Siliciumpulver nach einem der Ansprüche 1 bis 7, indem
a) mindestens eine bei erhöhter Temperatur gasförmige Siliciumverbindung,
b) optional in Gegenwart eines Verdünnungsgases in einer im Wesentlichen sauerstofffreien Atmosphäre unter (i) thermischen Bedingungen und/oder (ii) im Plasma zersetzt wird, und in Form eines Siliciumpulvers umfassend Submicropartikel deren Partikelgröße von 5 nm bis 3000 nm liegen, erhalten wird, wobei die Submicropartikel im Wesentlichen kristallin sind.

9. Verfahren nach Anspruch 8, **dadurch gekennzeichnet, dass** die bei erhöhter Temperatur gasförmige Siliciumverbindung umfasst Wasserstoff- und/oder Halogen-enthaltende Silane und/oder Kohlenwasserstoff-enthaltende Silane, wie Halogensilane, Chlorsilane, wie Dichlorsilan, Trichlorsilan, Tetrachlorsilan, Hexachlordisilan, Methyltrichlorsilan, Polyhalogensilane sowie reine H-Silane wie Monosilan, Wasserstoff enthaltende Polysilane oder Polyhalogensilane und/oder mindestens ein Alkoxysilan.

10. Verfahren nach Anspruch 8 oder 9, **dadurch gekennzeichnet, dass** das Verdünnungsgas Argon, Helium, Xenon, Krypton, Wasserstoff oder ein Gemisch mindestens zwei der genannten Gase umfasst.

11. Verfahren nach einem der Ansprüche 8 bis 10, **dadurch gekennzeichnet, dass** optional ein bei erhöhter Temperatur reaktives Gas umfassend eine Bor enthaltende Verbindung, wie Diboran, Stickstoff enthaltende Verbindung zugeführt wird, wie Stickstoff, NH₃, Alkylamine, Germanium enthaltende Verbindungen, Selen enthaltende Verbindungen, Kohlenwasserstoffe, wie Methan, Butan und/oder Propan, Verbindungen der vorgenannten Elemente (reaktive Gase) der dritten Hauptgruppe des Periodensystems der Elemente (Gruppe 13 PSE IUPAC), wie Aluminium, und/oder Elementen der fünften Hauptgruppe (15 PSE IUPAC), wie Phosphor, Arsen, Antimon.

12. Verfahren nach einem der Ansprüche 8 bis 11, **dadurch gekennzeichnet, dass** die Verfahrensführung mindestens die Alternativen umfasst
- die (A) Zersetzung der Siliciumverbindung in Gegenwart eines Verdünnungsgases und optional mindestens eines reaktiven Gases unter (i) thermischen Bedingungen und/oder (ii) im Plasma, und Ausbildung von Submicropartikeln und/oder Macropartikeln in Form von Clustern der Submicropartikel, und optional Ausbildung mindestens einer Zone einer Siliciumlegierung, mit den Schritten
- Ausbildung (B) der nanokristallinen, reinen Silicium enthaltenden Submicropartikel und/oder Macropartikel unter (i) thermischen Bedingungen und/oder in (ii) einer Behandlung im Plasma und optional Ausbildung der genannten Partikel umfassend eine Siliciumlegierung und/oder
- Ausbildung mindestens einer Zone einer Siliciumlegierung (C) auf gebildeten Submicropartikeln und/oder Macropartikeln, unter (i) thermischen Bedingungen und/oder in (ii) einer Behandlung im Plasma.

13. Verfahren nach einem der Ansprüche 8 bis 12, **dadurch gekennzeichnet, dass** das Siliciumpulver gebildet wird, durch
1) Zersetzen der Siliciumverbindung,
2) Abkühlen des gasförmigen Siliciums, insbesondere in Gegenwart inerter Verdünnungsgase und/oder
3) das Siliciumpulver gebildet wird durch Inkontaktbringen der zersetzten Siliciumverbindung mit einem kühleren reaktiven Gas unter Bildung und Abkühlen des beschichteten Siliciumpulvers und Abscheidung des Pulvers.

14. Siliciumpulver erhältlich nach einem Verfahren nach einem der Ansprüche 8 bis 11, **dadurch gekennzeichnet, dass** die Siliciumpulver Submicropartikel umfasst und die Submicropartikel eine Partikelgröße von 5 bis 3000 nm aufweisen, wobei die Submicropartikel im Wesentlichen kristallin sind und, wobei das Siliciumpulver eine Kristallinität von größer gleich 15,5 % bis kleiner gleich 99,9 % aufweist.

15. Reaktor (Free-Space Reaktors) (1) zur Herstellung von Siliciumpulvern nach einem der Ansprüche 1 bis 13, mit Submicropartikeln deren Partikelgröße von 5 nm bis 3000 nm liegt, wobei der Reaktor (1) einen Reaktionszylinder (2) mit Reaktionsraum (2.1) umfasst und im Bereich des Reaktoranfangs (2.a, 3) mindestens eine Zuführung (4.1, 4.2, 4.3) aufweist für die Einleitung a) der mindestens einen bei erhöhter Temperatur gasförmigen Siliciumverbindung, und b) optional des Verdünnungsgases, in den Reaktionsraum (2.1), wobei dem Reaktionsraum (2.1) zumindest in einem Bereich am äußeren Umfang des Reaktionsraumes (2.1) Heizvorrichtungen (7) zugeordnet sind, und/oder dem Reaktionsraum (2.1) mindestens ein Plasmagenerator (9) zugeordnet ist, zur Erzeugung eines Plasmas im Reaktionsraum (2.1).

16. Verwendung der Siliciumpulver nach einem der Ansprüche 1 bis 7 oder erhältlich nach einem Verfahren nach einem der Ansprüche 8 bis 13, zur Herstellung von Solarsilicium, Halbleitersilicium, Thermoelementen, Thermoelementen zur Energierückgewinnung aus Abwärme, thermoelektrischen Komponenten, wie thermoelektrischen Generatoren (TEG), Halbleitern, Halbleitermaterialien, Pasten, Cremes, Kosmetikprodukten, pharmazeutischen Produkten, Silicium enthaltenden Pasten, Elektroden, Membranen, Katalysatoren, von LEDs, Displays, transparenter Schichten, Flat Panel Displays, Schmelztiegeln, Tiegeln, als Ausgangsverbindung zur Kristallisation hochreiner metallischer Silicium-Verbindungen, Beschichtungen, Aerosolen, Materialien für die Thermoelektrik, als Ausgangsverbindung zur Herstellung hochreiner polykristalliner Metallverbindungen oder von Einkristallen der Metallverbindungen, in Hochtemperatur Transistoren, die bei 100 bis 400 °C, vorzugsweise um 300 °C betrieben werden können, zur Herstellung von Hochtemperatursperrschichten, die bei 300 bis 400 °C betrieben werden können, zur Herstellung von Hochtemperaturmembranen, die bei 300 bis 400 °C betrieben werden können, zur Herstellung von Hochtemperaturbauteilen in Batterien, die bei 300 bis 400 °C betrieben werden können, zur Herstellung von Bauteilen, deren Absorption von 300 bis 1000 nm sich im Wesentlichen linear ändert, insbesondere von Bauteilen, deren Transmission bei etwa 1000 nm 50% beträgt und bis 450 nm oder bis etwa 300 nm 20% beträgt und ab 300 nm im Wesentlichen keine Transmission mehr erlaubt, oder als Reaktand bei der Herstellung von kristallinem Silicium oder kristallinen Siliciumverbindungen, Herstellung von OLED, hochreine Schleifmittel, hochreine Hitzekacheln oder als Material von Artikeln, zur Herstellung von Beschichtungen von Tiegeln (Crucibles).
